# EUROPEAN PATENT SPECIFICATION

(11) **EP 3 057 652 B1**
(45) Date of publication and mention of the grant of the patent: **12.07.2023**
(21) Application number: 14853504.0
(22) Date of filing: 17.10.2014
(51) Int. Cl.: A61N 1/36, A61N 1/05, A61N 1/375

(54) **DEVICES FOR STIMULATING NERVES**
VORRICHTUNGEN ZUR STIMULIERUNG VON NERVEN
DISPOSITIFS DE STIMULATION DES NERFS

(30) Priority: 17.10.2013 US 201314056937; 09.12.2013 US 201314101336
(43) Date of publication of application: 24.08.2016
(73) Proprietor: Fempulse, LLC, Incline Village, NV 89451 (US); Haessler, Alexandra, Larkspur, CA 94977 (US); Strul, Bruno, Portola Valley, CA 94028 (US)
(72) Inventor: HAESSLER, Alexandra, Larkspur, CA 94977 (US); STRUL, Bruno, Portola Valley, CA 94028 (US)
(74) Representative: Schaafhausen Patentanwälte PartGmbB
(86) International application number: PCT/US2014/061209
(87) International publication number: WO 2015/058128

(56) References cited:
- WO-A1-99/22880
- US-A- 4 771 779
- US-A- 4 827 946
- US-A- 4 881 526
- US-A- 4 955 378
- US-A- 5 370 671
- US-A1- 2004 006 282
- US-A1- 2005 256 423
- US-A1- 2007 142 748
- US-A1- 2008 171 950
- US-A1- 2008 177 398
- US-A1- 2008 300 850
- US-A1- 2009 228 064
- US-A1- 2009 228 064
- US-A1- 2011 202 108
- US-A1- 2012 245 852
- US-A1- 2013 131 772
- US-A1- 2013 144 191
- US-A1- 2013 204 328
- US-B1- 6 185 465
- US-B1- 6 445 955
- US-B1- 6 625 495
- US-B1- 6 741 895
- US-B1- 6 741 895
- US-B2- 6 526 960
- US-B2- 6 905 471
- US-B2- 7 328 068
- US-B2- 8 114 610
- US-B2- 8 165 692
- US-B2- 8 452 407
- US-B2- 8 467 875
- US-B2- 8 788 040

## Description

### BACKGROUND

Millions of women suffer from increased urinary urgency, frequency, incontinence, incomplete bladder emptying and irritative bladder conditions. There are two main types of incontinence, urge incontinence and stress incontinence, which have different physiological causes and different treatment options. Many women suffer from mixed incontinence, having both conditions.

Stress incontinence is largely the result of weakened ligaments, pelvic floor tissue and vaginal support, ultimately allowing the urethra to drop open in the setting of increased intraabdominal pressure. The process leading to stress incontinence is primarily mechanical and is associated with supportive tissue under the distal anterior vaginal wall. Strengthening the pelvic floor and supporting the urethra and/or bladder neck are safe and effective treatments for stress incontinence.

Urinary urgency, frequency, urge incontinence, incomplete bladder emptying, and nocturia represent a more complex process that is greatly neurologically mediated. Urinary urgency is marked by a strong, and often uncomfortable, urge to void that is difficult to suppress. Urge incontinence is defined by involuntary leakage of urine in the setting of urgency. The conditions noted above and other irritative bladder conditions may result from abnormal sensitivity of autonomic bladder nerves that prematurely communicate "fullness" to the central nervous system. These conditions may also result from erroneous nerve function in the central nervous system that allows the bladder to empty at inappropriate times. The underlying cause of these problems may be multifactorial. Ultimately, it is the autonomic nervous system that communicates bladder sensation, discomfort, or "fullness" and coordinates bladder filling and emptying.

Incomplete bladder emptying or urinary retention may result from inappropriate autonomic nerve signaling to the urethra and / or bladder. Some medical treatments are aimed at altering autonomic signal to the urethra, such that urethral tone is lessened. Others work by increasing bladder muscle tone by activating autonomic receptors in the bladder to cause a bladder contraction.

Many treatments of irritative bladder conditions are aimed at modifying nerve signaling between the bladder and the central nervous system. The goal is to affect both the afferent signals to the central nervous system that signal irritation, urgency, pressure or a sense of fullness and/or the efferent nerve signal that can trigger a rise in bladder pressure, cause discomfort, or leakage. Autonomic nerves transmit these signals. Treatment for urinary urgency, frequency, nocturia and urge incontinence is multifaceted, attempting to affect nerve signaling with behavioral, dietary, medical and physical therapies.

Urinary urge incontinence medications attempt to inhibit bladder contractions by disrupting signals between the autonomic nerves and the urinary tract. These drugs are not adequately effective for the majority of patients and also have significant systemic side effects that limit usage. Discontinuation rates at 6 months are well over 50% due to the high cost, lack of adequate effectiveness, and side effects. There are also contraindications to use, most of which are conditions affecting the elderly population that desires treatment for lower urinary tract symptoms.

An aggressive pelvic floor physical therapy regimen incorporating education, lifestyle, and dietary changes along with pelvic floor exercise training can be a reasonably effective treatment for urgency, frequency and urge incontinence in some patients. It is not clear how well exercise alone works to control urge related symptoms, as a large portion of the benefit gained from physical therapy is the result of education, cognitive-behavioral techniques, diet and lifestyle change. Patients are not often compliant beyond the short term, as the regimen can be costly, time consuming and difficult to comply with over time.

The goal of most pelvic floor exercises is to strengthen the muscles of the pelvic floor and increase nerve "tone." These exercises activate the somatic nerves in order to cause pelvic floor muscle contraction. This is primarily a treatment for stress incontinence. A pelvic floor muscle contraction can secondarily affect autonomic nerves to the bladder via a spinal cord reflex arc. These exercises may dampen the signal to urinate or prevent leakage. They do not necessarily prevent the more common overactive bladder symptoms that occur before one has time to perform a contraction, such as a spontaneous sense of urgency or waking at night to void. It has been shown that urinary urgency alone, without leakage, is more bothersome to patients than urinary leakage alone. Effective treatment needs to prevent the spinal cord from perceiving inappropriate sensations in the first place.

Neuromodulation of autonomic nerves has become a useful treatment method for lower urinary tract symptoms, such as urgency, frequency, incontinence, incomplete bladder emptying, irritative symptoms, incomplete bladder emptying, fecal incontinence and related conditions. One technique involves an implanted stimulation lead over the S3 nerve root. It can be a successful treatment for patients who have failed other treatments.

Although use of the surgical implant over the S3 nerve root has been successful, the treatment has drawbacks. The main disadvantage is that the device requires surgical implantation of small electrical leads through a foramen in the sacrum and a stimulator device within the soft tissue above the gluteus maximus. Implantation typically requires two surgical procedures, exposure to radiation, and often requires additional surgery to revise or remove the components. It has also been reported that over 25% of patients will require surgical revision of either the implanted battery or lead to manage complications. Additionally, the current surgical implant often needs to be removed for some types of MRI. The device is also extremely expensive. In general, patients prefer less effective alternative therapies, as they often do not think bladder symptoms warrant surgery or they are not comfortable with the idea of a surgical implant.

Another drawback of the conventional surgical implant is that the stimulation occurs at the level of the nerve root, so somatic fibers traveling in the S3 nerve root are also stimulated before they branch off into somatic nerves. These nerves can cause the patients to feel vulvar, vaginal, anal and lower extremity muscle contractions or tingling. Still another limitation of the implantable stimulator is that the same one, or occasionally two, nerve roots are available and subject to all of the stimulation. As a result, the nerve becomes less responsive due to habituation rendering the lead less effective or ineffective. This phenomenon has been well established. Due to these many drawbacks, including risk of surgery, complications associated with the implant, cost, reduced effectiveness over time, and somatic symptoms associated with the implanted S3 sacral nerve modulator, this treatment has not been widely adopted despite its effectiveness.

The existing non-surgical electrical stimulation therapies include devices that are positioned in the vagina thereby avoiding some drawbacks of the surgical implant. However, these devices are often less effective and have their own drawbacks. Many conventional devices positioned in the vagina direct electrical impulses in the lower or distal vagina and/or adjacent to the pelvic floor. Electrical signals are sent through the vagina to paravaginal tissue, targeting nerves adj acent the pelvic floor muscles or the muscles themselves. The goal suggested by these conventional methods is generally to stimulate nerves adjacent the pelvic floor and to increase pelvic floor tension. Many of these therapies attempt to treat stress incontinence and/or simulate pelvic floor exercises. Yet, bladder filling, emptying and sensation are mediated by autonomic nerves, not somatic nerves. The inventor believes that these devices and therapies are often not as effective at treating urinary urgency, incontinence, nocturia, incomplete bladder emptying or other lower urinary tract symptoms as an implanted sacral nerve modulator because they do not sufficiently isolate stimulation to autonomic nerves.

A major limitation of these vaginal stimulation devices for treating most irritative urinary tract symptoms is that they indiscriminately send electrical impulses to both somatic and some distal autonomic nerve fibers adjacent to the lower and mid-vagina. They also often target somatic nerves, such as the pudendal nerve and its distal branches. The somatic nerves do not directly control bladder filling, emptying, or sensation. Activation of somatic nerves can cause increased pelvic floor tension, discomfort, and pain associated with pudendal nerve activation. Such conditions may actually contribute to urinary urgency, frequency and voiding dysfunction. Somatic nerves are quite sensitive, so the electrical impulses can be perceived even at low intensity, thereby limiting the full range of treatment protocols or precluding therapy altogether.

Thus, the inventor believes that the problem with present neuromodulation treatment methods is that they either require surgical implantation or, for vaginally inserted devices, the stimulation does not adequately target important autonomic nerve structures yet influences muscles and somatic nerves in the lower vagina providing less effective therapy for autonomically mediated urinary tract symptoms and contributing to undesirable side effects. Furthermore, the conventional implanted sacral nerve root therapies stimulate somatic nerves (although there are also autonomic - parasympathetic fibers near this location) and provide therapy to a very focal portion of a nerve, thereby contributing to habituation. These factors may provide less effective therapy for autonomically mediated urinary tract symptoms and contribute to undesirable side effects. Thus, optimal therapy would stimulate the autonomic nerves that mediate signal between the urinary tract and the central nervous system and/or end organ while avoiding activation of the pelvic floor and associated somatic nerves. Optimally, neuromodulation would be delivered directly to the autonomic nerves at the "gateway" between the urinary tract and the spinal cord while avoiding pelvic floor muscle and nerve activation.

The present invention is also directed to systems for altering and improving (reducing) sympathetic tone. The autonomic nervous system maintains homeostasis and functional control of most organs in the body. The sympathetic arm of the system mediates the "fight or flight" response associated with a stress state, generally via norepinephrine, otherwise known as "adrenaline." The sympathetic system is responsible for preparing animals for immediate action. The parasympathetic arm maintains organ function at rest. It is theorized that survival of early organisms was quite dependent on the ability to quickly adjust from rest (grazing) to extreme physical activity (fleeing a predator). This transition requires immediate coordination of many organs, glands and nerves that increase cardiac output, optimize respiratory function, increase skeletal muscle contractility, alter blood flow distribution, adjust visual depth perception, and trigger rapid-fire thinking. When the sympathetic tone is high, organs are operating at an extreme capacity, which is not healthy over time.

Outside of acute stress, organisms need to maintain a fine balance between the sympathetic and parasympathetic tone. In modern times humans are not often fleeing lifethreatening danger. However, we tend to live in a chronic state of stress. Naturally, the sympathetic nervous output is upregulated in a chronic stress state. Chronic stress contributes to persistently elevated sympathetic tone, which has deleterious effects on most organs of the body. For example, the heart is very sensitive to even modest increases in circulating epinephrine. Chronic exposure to increased epinephrine levels results in a rise of blood pressure and heart rate that can contribute to many forms of heart disease, including arrhythmias, coronary artery disease, valvular dysfunction and heart failure. Alterations in cerebral blood flow resulting from stress, influenced by the sympathetic nervous system, can result in migraine headaches.

Metabolism is greatly affected by increased sympathetic tone, via increased release of cortisol. Cortisol is an adrenal hormone that alters metabolism of glucose, bone, muscle, adipose (fat) cells and connective tissue. Cortisol acts locally and systemically to mobilize metabolic resources for a stress response related action. Chronically elevated circulating cortisol levels can have serious and deleterious consequences including insulin resistance/diabetes, decreased bone formation (osteoporosis), inhibited collagen production (poor tissue quality and wound healing), decreased protein synthesis (atrophy), increased gastric acid secretion (ulcers and gastrointestinal bleeding), altered renal function (electrolyte imbalances), impaired hippocampal function (decreased learning ability and dampened memory retrieval), along with increased epinephrine sensitivity in blood vessels (hypertension). Cortisol also weakens the immune response by inhibiting T cell proliferation, which results in vulnerability to infection and cancer.

Stress and elevated sympathetic tone is also associated with increases in circulating inflammatory mediators, mainly Interleukin-6, which promote systemic inflammation. Inflammation is responsible for autoimmune conditions affecting nearly every organ in the body. Examples include dermatologic conditions (eczema), atherosclerosis (coronary artery disease and stroke), gastrointestinal (ulcerative coltis or Crohn's disease), lupus, arthritis, allergic reactions, pericarditis, myocytis, cataracts, pain conditions, interstitial cystitis, vasculitis, glomerulopathies, multiple sclerosis, iritis, asthma, and cancer. Pain is associated with inflammation and stress. Increased mortality has been associated with chronic exposure to increased stress and Interleukin-6 levels.

Many people simply maintain an elevated sympathetic tone, regardless of their stress level. Normal blood pressure and heart rate in healthy and relaxed people varies within a normal range, for example. Central obesity contributes to increased sympathetic output, regardless of stress level or cardiovascular health.

The role of autonomic imbalance in leading to poor cardiovascular health, and in causing heart failure, is increasingly being recognized. Research shows elevated inflammation in heart failure patients, with inflammation linked to left ventricular dysfunction. Poor autonomic tone is related to arrhythmia and increased heart rate.

The current approaches to delivering autonomic modulation are based on implanted devices. Even monitoring devices for recording heart rhythms that do not deliver stimulation, are often implanted under the skin, requiring a procedure. By contrast, the present invention provides for both monitoring of heart rhythms (rate and waveform) and delivery of autonomic neuromodulation by vaginally-inserted and self-retained systems and methods, complemented by wireless transmission of data from a user' s existing personal device which may act as a controller.

Ultimately, lowering sympathetic tone can improve specific disease states and overall health. Current approaches to decrease sympathetic tone include exercise, weight loss, relaxation techniques, meditation and cognitive behavioral therapies. These approaches are not always effective, achievable or sustainable.

The present invention is directed to improved systems for stimulating nerves. The present invention is also specifically directed to systems for altering sympathetic tone. The present invention allows monitoring and sensing, diagnosis, and/or therapeutic treatment of numerous medical conditions.

US 2008/171950 A1 discloses systems and methods for characterizing electrical activity of a patient for making a pregnancy-related diagnosis. The system includes a wearable device for measuring an electrical impedance of a cervical tissue of the patient based in a signal applied to the cervical surface by the device. The system also includes a transmitter coupled to the wearable device for transmitting the measured electrical impedance of the cervical tissue to an analyses system for making a pregnancy-related diagnosis based in the impedance data

US 2004/006282 A1 discloses two electrodes for contacting consistently a selected position in the vaginal fornix of a mammalian female such as a cow which are brought to the desired position so as to allow daily measurements indicative of the follicular waves and then of the optimal time for insemination. The detection of the follicular waves makes it possible to anticipate optimal breeding time first by 10 days and then by 3 days. The monitoring of the animal's reproductive cycle consists of daily measurements of admittance or small AC current generated by time-varying voltage of small amplitude and sufficiently high frequency.

### SUMMARY

The invention is defined by claim 1. Preferred embodiments are defined by the dependent claims. Further embodiments, aspects and examples disclosed herein are for exemplary purposes only and do not form part of the claimed invention.

The current invention will decrease sympathetic tone, thereby protecting organs (such as the heart and vascular system) from deleterious effects of chronically increased sympathetic stimulation (via hormones, neurotransmitters, chemicals) and/or mitigate systemic disease processes influenced by sympathetic outflow, such as inflammation and immunosuppression.

The present invention provides an implant with a nerve stimulating element which is implanted and preferably secured to a uterosacral ligaments. The implant modulates the action of nerves and treats conditions by altering the signals of autonomic nerve plexuses, their associated nerves and nerve endings and potentially interneurons and central nervous systems nerves and processes via one or more nerve stimulating implants. The implant may be used independently, together with other implants, or in combination with the vaginally positioned devices described herein. The vaginal device may also stimulate the anatomic structures described either by creating a circuit with the implanted electrodes or independently.

The present invention treats urologic, gynecologic, sexual, colorectal, and pain conditions in women with neuromodulation via electrical stimulation (and other modalities), (including heat, ultrasound, and medication). The implant delivers therapy to specific autonomic plexuses that communicate with nerves traveling to and from genitourinary and pelvic structures. The neuromodulation of the present invention stimulates the nerve plexuses to alter signal transmitted by the nerve plexuses and associated autonomic nerves between the pelvic organs and the central nervous system.

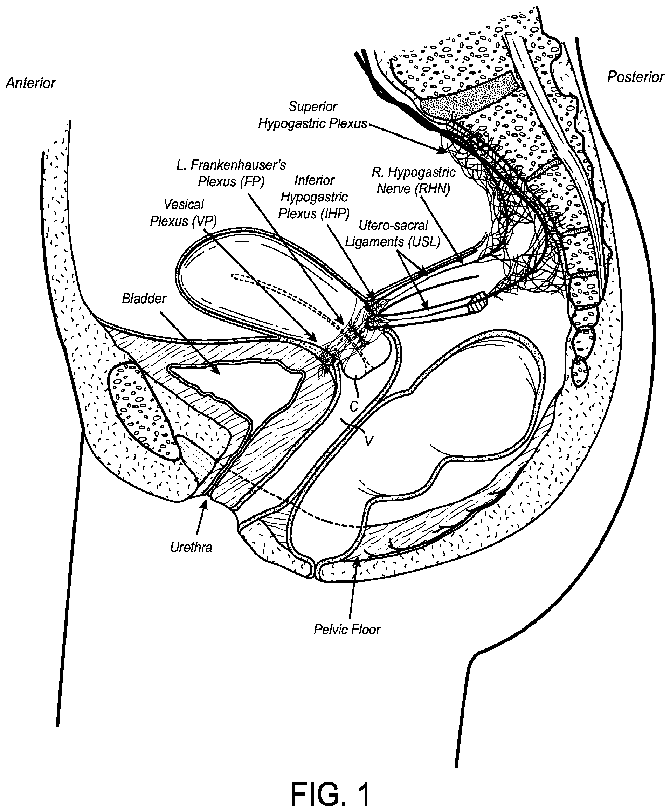

For reference (Fig. 1) autonomic nerve signals traveling to and from the bladder, urethra, rectum, vagina, uterus, peritoneum and other pelvic structures travel through the inferior hypogastric plexus IHP, carrying the signal between pelvic structures and the spinal cord. The inferior hypogastric plexus IHP is the gateway between the visceral (bladder, gynecologic, rectum) organs and the central nervous system. The IHP is a coalescence of both sympathetic and parasympathetic autonomic fibers. The sympathetic fibers run between the thoracic nerve roots (TIO-L2) and into the superior hypogastric plexus, then travel towards the IHP via the left and right hypogastric nerves LHN, RHN along the uterosacral ligaments USL. In some cases a portion of the sympathetic fibers may enter the IHP along the undersurface of the cardinal ligaments. These fibers facilitate bladder storage. The parasympathetic nerves travel from the sacral spinal nerve roots (S2-4) in the sacral or pelvic splanchnic nerves, or nervi erigentes towards the pelvic organs, often in association with the pelvic nerve. They facilitate bladder emptying. Sympathetic and parasympathetic fibers coalesce in the IHP over the posterior and lateral surfaces of the upper cervix, just above the vaginal insertion.

Importantly, the vagina terminates at the level of the pericervical ring (known by other names) around the uppermost portion of the cervix, just below the uterosacral ligament USL insertions. The recesses of the most proximal, or uppermost, aspect of the vagina that surround the cervix are called the vaginal fornices VF. The pelvic floor PF surrounds the lower portion of the vagina near the vaginal opening, making the "floor" of the pelvic cavity. The inferior hypogastric plexus IHP is a web-like plexus adjacent to the posterior and lateral cervix at the level of the uterocervical junction and the uterosacral ligament USL insertions, just proximal to the posterior vaginal fornix. Inferior hypogastric plexus IHP fibers also cover the distal uterosacral ligaments and possibly reside in the lower or under-portion of the cardinal ligament just anterior to the USL. For reference, the IHP is generally referred to as a singular structure in the literature, but anatomic studies suggest significant concentrations of these ganglia may be distributed bilaterally, as described above. For purposes of this description and application the IHP, when used herein, will refer to the IHP and any lateral extensions around the cervix or most proximal aspect of the vagina.

Autonomic nerve fibers travel from the IHP around the cervix to the vesical plexus, which communicates with autonomic fibers to innervate the bladder. Some autonomic fibers travel from the IHP to the posterior-lateral aspect of the cervix to right and left Frankenhauser's plexuses FP (also known as Lee's or the uterovaginal plexuses). Frankenhauser's or Lee's plexuses send some nerves upward to innervate the uterus and some nerves inferiorly to innervate the vagina, cervix, urethra, and clitoris. Both of these plexuses may be important in controlling urinary tract function. Autonomic fibers travel between the IHP and the middle rectal plexus to innervate the distal rectum.

In one embodiment of the present invention, the implant has a nerve stimulating element, such as an electrode, which is positioned around and/or through one or both uterosacral ligaments, adjacent to the inferior hypogastric plexus, Frankenhauser' s plexus or the superior hypogastric plexus. The implant may also be secured to other structures, such as pubocervical fascia or the external vaginal wall, without departing from the scope of the invention. The therapy is designed to also treat the autonomic nerves traveling around the pericervical and apical vaginal fascia / connective tissue to visceral pelvic organs and the vesicle plexuses,

In one embodiment, the implant is generally cylindrical in shape with tapered ends. The implant may include a throughhole to accommodate the uterosacral ligament. The nerve stimulating element may be between 0.5 and 5.0 cm long and up to 2 cm wide. The outer surface of each implant is made of a biocompatible and insulating material to which the nerve stimulating elements are attached. The implant has a power source, such as a battery, which powers the implant. The battery may be rechargable and recharged remotely through tissue as is known in the art. For example, the charger may be positioned in the vagina and, in particular, positioned within the vaginal fornices VF.

The size and shape of the device is designed to provide an optimal surface area for stimulation of an autonomic plexus and associated nerves. The nerve stimulating element may be a relatively small microstimulator when treating a somatic nerve or nerve junction, such as a dorsal nerve ganglion. Autonomic nerves plexuses, on the other hand, are broader, so the nerves coalesce more diffusely thereby possibly necessitating a larger nerve stimulator. Broader distribution of therapy may be important for treatment success.

The uterosacral ligaments contain autonomic nerves as they travel to and from plexuses from the visceral pelvic organs. The implant may have two nerve stimulating elements (such as electrodes) in contact with the uterosacral ligament and positioned in series along the ligament.

The systems of the present invention stimulate target nerves and plexuses while avoiding adversely influencing non-target somatic nerves, such as the pudendal nerve or its branches, adjacent to the distal or lower half of the vagina. Reducing side effects and possible urinary tract symptoms due to stimulating somatic nerves are thereby avoided in accordance with the present invention.

In addition to other treatments describe herein, the present invention may be used to treat any one or more of the following conditions: urinary urgency, frequency, nocturia, urge incontinence, stress incontinence of urine, loss of urine without sensory awareness, overflow incontinence, bladder pain, urethral pain, urethral syndrome, urethral stricture, urinary hesitancy, protracted urinary stream, pelvic floor dyssynergia, interstitial cystitis, dysuria, overactive bladder, incomplete bladder emptying, urinary retention, hesitancy, dysmenorrhea, pelvic pain, pelvic venous congestion syndrome, endometriosis, irritable bowel syndrome, constipation, fecal urgency, fecal incontinence, rectal pain, pain with defecation, anal pain, sexual dysfunction, and abnormal heart rhythm. Of course, numerous aspects of the present invention may be practiced for a different condition without departing from the scope of the invention.

The details of one or more embodiments of the invention are set forth in the accompanying drawings and the description below. Other features, objects, and advantages of the invention will be apparent from the description and drawings, and from the claims.

### DESCRIPTION OF DRAWINGS

Figure I is a parasagital view into the female pelvis from the left.
Figure 2 is an enlarged view of the upper vagina and uterus.
Figure 3 is a posterior view of the gynecologic organs.
Figure 4 is an enlarged view of the upper vagina and uterus with the device of the present invention positioned in the vaginal fornices.
Figure 5 is a posterior view of the gynecologic organs with the device positioned in the vaginal fornices.
Figure 6 shows the device, a charger and a controller.
Figure 7 shows removal of the device from the charger.
Figure 8 is a top view of the device showing the electrodes.
Figure 9 shows the orientation of the electrodes.
Figure 10 shows a battery, control system and hub, which interconnects the control system with the electrodes.
Figure Il is an exploded view of the hub, battery and control system.
Figure 12 shows the hub connected to the battery and control system.
Figure 13 shows a layer applied over the main body to seal the battery and control system within the main body.
Figure 14A is a cross-sectional view of the main body.
Figure 14B is another cross-sectional view of the main body with an alternative electrode.
Figure 15A is a perspective view of one end of the main body.
Figure 15B is a perspective view of the same end of the main body as Figure 15A with the alternative electrode of Figure 14B.
Figure 16 is a perspective view of the end of the main body with the battery positioned in the main body.
Figure 17 is a perspective view of the end of the main body with the control system positioned in the main body.
Figure 18 shows an end of the hub that abuts against the battery.
Figure 19 shows another end of the hub that is coupled to the control system.
Figure 20 shows a top view of another device in accordance with the present invention.
Figure 21 shows still another device in accordance with the present invention.
Figure 22 shows another device for stimulating nerves.
Figure 23 shows another device for stimulating nerves for use with the device of Fig. 22.
Figure 24 shows another device for stimulating nerves.
Figure 25 shows another device for stimulating nerves with the nerve stimulating element mounted to a movable element on the main body of the device.
Figure 26 shows the movable elements of Fig. 25 moved together.
Figure 27 shows a first cover and a second cover for covering parts of the devices described herein.
Figure 28 shows another device for stimulating nerves.
Figure 29 shows a cross section of the device of Figure 28.
Figure 30 shows an enlarged view of a recess of the device of Figure 28.
Figure 31 shows the recess filed with a porous material, which holds an electrically conductive substance.
Figure 32 shows another device for stimulating nerves.
Figure 33 shows still another device for stimulating nerves.
Figure 34 shows the device of Figure 33 with the recess filled with an electrically conductive, pliable material.
Figure 35 shows the nerve stimulating element covered by an electrically conductive material.
Figure 36 shows another device for stimulating nerves having an insert with conductive and non-conductive regions.
Figure 37 is a plan view of another device.
Figure 38 is a bottom view of the insert used with the device of Figure 37.
Figure 39 is a top view of the insert of Figure 37.
Figure 40 is a partial cross-sectional simplified view showing the nerve stimulating element positioned in the recess.
Figure 41 is a partial cross-sectional simplified view showing the nerve stimulating element positioned in the recess with the conductive material positioned over the nerve stimulating element.
Figure 42 shows a dissolvable portion forming part of the main body of the device of Figure 37.
Figure 43 is a plan view of the device with the dissolvable portion removed.
Figure 44 shows a device having a torus-shaped exterior surface with a wall positioned near the central opening.
Figure 45 shows a device having a torus-shaped exterior surface with the nerve stimulating element positioned in a recess.
Figure 46 is a simplified cross-sectional view showing the nerve stimulating element of Figure 44 contained in a recess.
Figure 47 is a simplified cross-sectional view showing the nerve stimulating element of Figure 45 contained in a recess.
Figure 48 is an alternative cross-sectional view for Figure 47.
Figure 49 shows still another device for stimulating nerves having a torus shaped exterior surface with recesses forming a scalloped appearance.
Figure 50 shows a simplified cross-sectional view showing the nerve stimulating element of Figure 44 contained in a recess.
Figure 51 shows an insert that may be coupled to any of the devices described herein.
Figure 52 shows an implant.
Figure 53 shows another implant.
Figure 54 shows a delivery tool holding the implant with the implant in an open position for introduction of the uterosacral ligament.
Figure 55 shows a cross-sectional view of the abdomen and pelvic region.
Figure 56 shows a window formed in the peritoneum.
Figure 57 shows a sizer introduced into the abdoment with the uterosacral ligament elevated.
Figure 58 shows the sizer extending around the ligament to size the ligament.
Figure 59 shows the delivery tool with the implant approaching the ligament.
Figure 60 shows the implant secured to the ligament.
Figure 61 shows the delivery tool removed and implant secured to the ligament.
Figure 62 shows another implant secured to the other uterosacral ligament.
Figure 63 shows another view of the window formed in the peritoneum.
Figure 64 is another view of showing the opening formed in the peritoneum.
Figure 65 is another view of the ligament being sized.
Figure 66 is another view of the delivery tool introducing the implant into the abdomen.
Figure 67 shows the implant positioned around the ligament with the delivery tool.
Figure 68 shows an implant secured to each uterosacral ligament.
Figure 69 shows the device positioned with the vaginal fornices and two implants.
Figure 70 shows two implants secured to each uterosacral ligament.
Figure 71 shows an exploded view of another implant.
Figure 72 shows the fully constructed implant of Figure 71.
Figure 73 shows another device, which may be positioned in the vagina to stimulate nerves.
Figure 74 shows the device of Figure 73 together with a controller and battery charger for the device.
Figure 75 shows a device for charging a power source for an implant of the present invention.
Figure 76 shows another device for charging a power source for an implant of the present invention.
Figure 77 shows the device of Figure 76 positioned in the vagina to charge the power sources for two implants each attached to a uterosacral ligament. Figure 78 shows another device for positioning in the vagina.
Figure 79 is a side view of the device of Fig. 81.
Figure 80 is a bottom cut-away view of the internal components of the device of Figure 81.
Figure 81 is another side view of the device of Figs. 78-80.
Figure 82 is a side view showing the main body manually bent for introduction and placement.
Figure 83 shows the device manually bent for introduction and a controller worn on the wrist.

### DETAILED DESCRIPTION

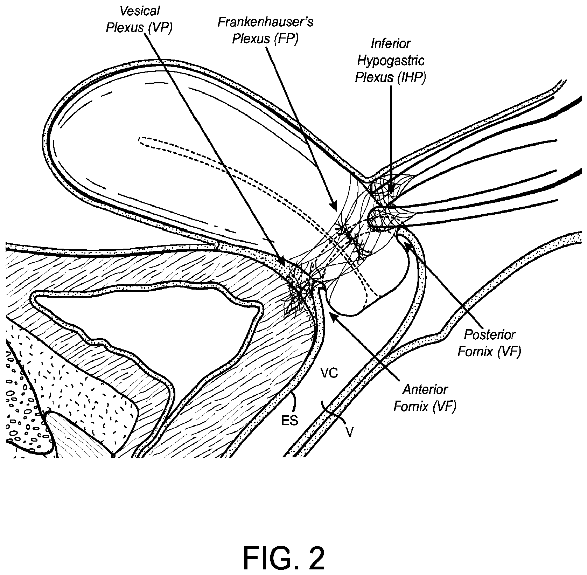

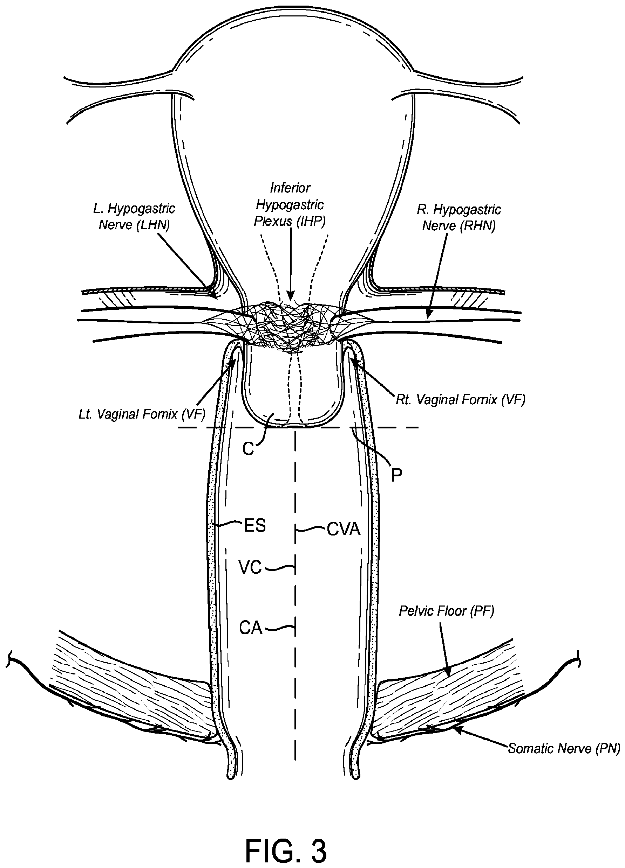

For the purpose of defining the invention, various terms are now defined for use herein with reference to Figs 1-3. Firstly, the vagina V is a fibroelastic, muscular structure that forms a canal (hereinafter "vaginal canal" VC) having a distensible, flexible lining. The surface of this flexible lining has an internal or exposed surface ES. The vagina, and vaginal canal, as defined herein has a proximal end terminating at the proximal aspect of the cervix and a distal end at the introitus where it joins the vulva. If the cervix C has been removed or is congenitally absent, the proximal end of the vagina (and vaginal canal) is simply defined by the uppermost portion of the vagina, also known as the vaginal cuff.

As used herein, the shape of the vaginal canal VC defines a central axis CA. A cervical axis CVA extends through and is aligned with the cervical canal CC. The cervical axis CVA is also an axis of symmetry, as closely as can be approximated with the anatomy, for the cervix extending through the cervical opening or cervical canal. A midpoint between the proximal and distal ends of the vagina (or the vaginal canal) is determined herein using the midpoint of the central axis. The central axis CA may, of course, have a relatively complex shape so long as the central axis CA generally defines and follows the orientation and shape of the vaginal canal VC. The proximal end of the vagina (and vaginal canal) is defined by the uppermost portion of the vagina. For example, a proximal portion of the vagina or vaginal canal extending 3 cm from the proximal end of the vagina includes the vagina extending 3 cm from the uppermost (proximal) end.

The vaginal fornices VF, as used herein, refers to a space or recess between the cervix and the adjacent vaginal wall. Specifically, the space is positioned between the exposed surface ES of the vaginal canal VC and an exposed surface of the cervix C and further bounded by a plane P extending perpendicular to the cervical axis CVA and passing through distal end of the cervix C. The vaginal fornices VF may be thought of as a somewhat torus-shaped space but, of course, varies from patient to patient.

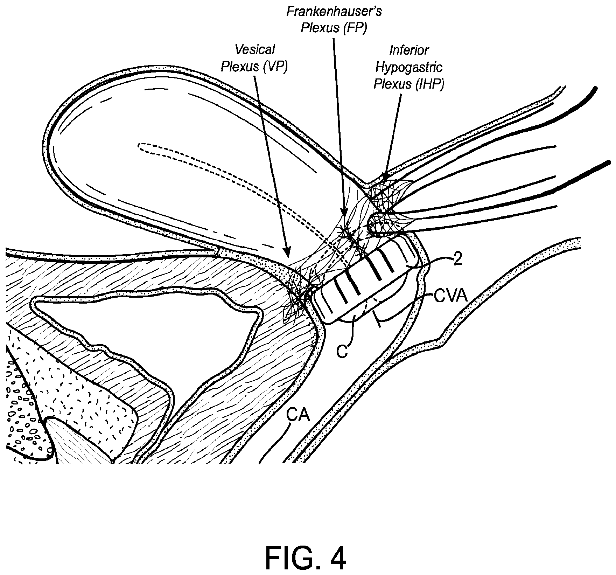

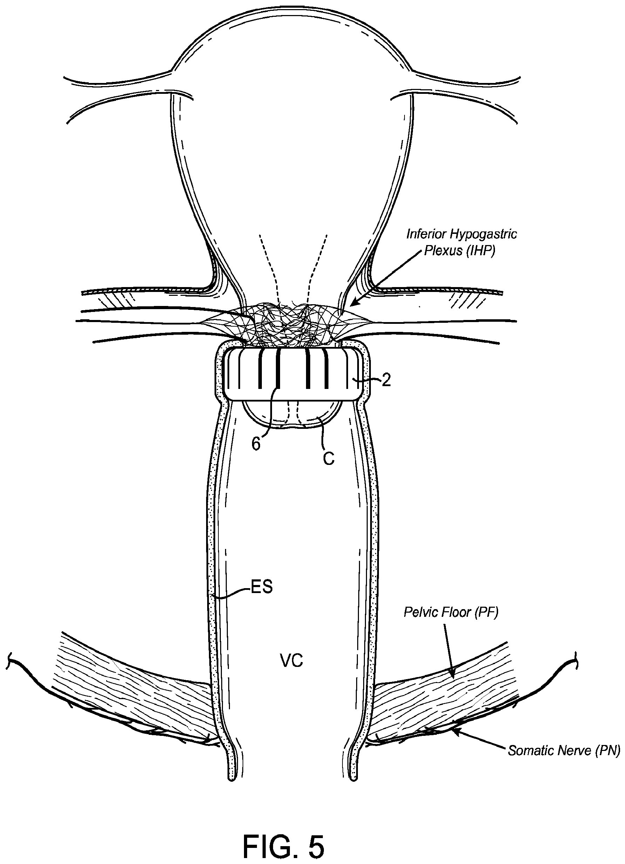

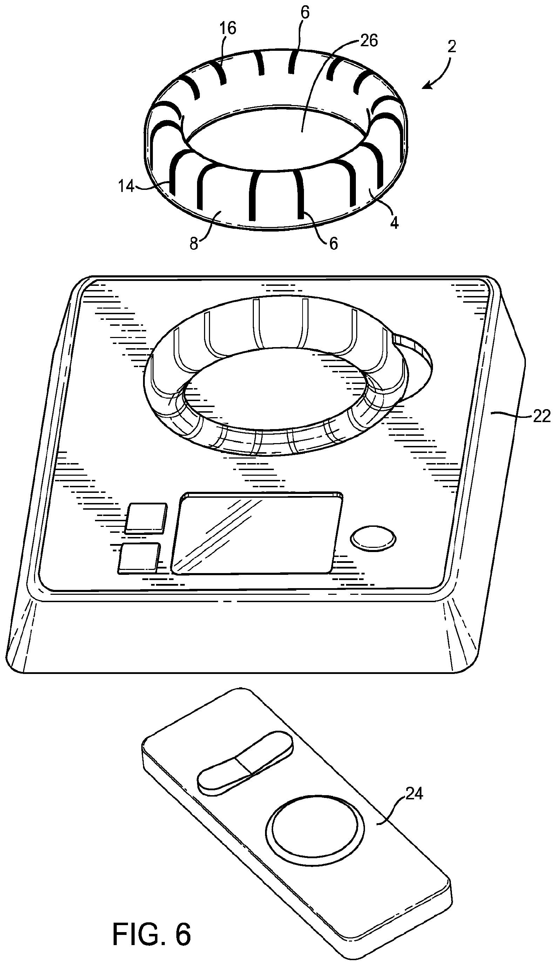

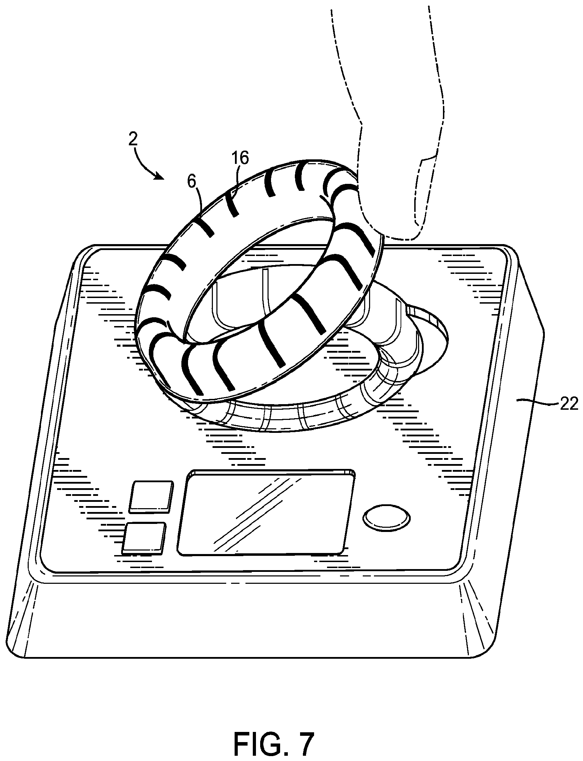

Referring now to Figs. 4-8, a device 2 in accordance with the present invention is shown. The device 2 includes a main body 4 having a plurality of nerve stimulating elements 6 positioned on an exterior surface 8 of the main body 4 for contact with the exposed or internal surface ES of the vaginal wall 12. The nerve stimulating elements 6 may stimulate nerves in a number of different ways without departing from numerous aspects of the invention. For example, the nerve stimulating element 6 may include one or more emitting elements 14 which emit electrical energy, ultrasound energy, a drug, a magnetic field or other suitable stimulus to the target nerves.

In a specific embodiment, the nerve stimulating elements 6 may be one or more electrodes 16 that deliver electrical energy to stimulate adjacent nerves as described below. As used herein, it is understood that use of "electrode" or "emitting element" herein shall be interchangeable with the term nerve stimulating element, and vice versa, as applicable. Thus, aspects of the invention described or claimed specifically in relation to the electrodes 16 or nerve stimulating element 6 are equally applicable to the other and such substitution is expressly incorporated here. Furthermore, the application of electrical stimulus is sufficient to change the signals transmitted by the target nerves and plexuses described below. In this manner, neuromodulation of the target nerves is achieved.

Similarly, aspects of the invention described or claimed in relation to electrodes or nerve stimulating elements will encompass sensing electrodes and related sensing elements, without distinguishing whether electrodes are emitting electrodes or sensing electrodes, nor whether a sensor is integrated with or simply communicates with a nerve stimulating element. In order to determine whether and how much and/or when to deliver neuromodulation or other therapysuch information coming from the nerve stimulating element itself as well as from sensors will be described in greater detail below.

The electrodes 16 are coupled to a control system 18 which in turn is coupled to a power source 19 such as a battery 20. The device 2 may include a battery charger 22 or may be charged transcutaneously as is known in the art. Of course, an advantage of the present invention is that the device 2 may easily be removed, charged and repositioned which cannot be accomplished with conventional surgically placed devices. The device 2 may also include an external controller 24 which may operate the device remotely when the device 2 is in place. The battery charger 22 may also be a controller for programming the device 2 in accordance with methods described herein. A few prior art devices which describe suitable control systems, controllers and battery charging systems are described in US Patent Nos. 7729772, 7813809 and 8165692. The controller 24 (and charger 22) may control the duration, frequency, intensity and stimulation protocol as described herein and in the patents incorporated above. The controller 24 may also be incorporated or applied to another suitable personal electronic device, such as a cellphone, programmable watch, tablet, laptop, electronic calendar/organizer or any other suitable personal programmable electronic device, without departing from the scope of the invention. If the controller 24 is incorporated into a personal electronic device the necessary control software can be downloaded and updated as a conventional application. The controller 24 may take advantage of the ability of the personal electronic device to communicate wirelessly with the device 2 (and other devices described below).

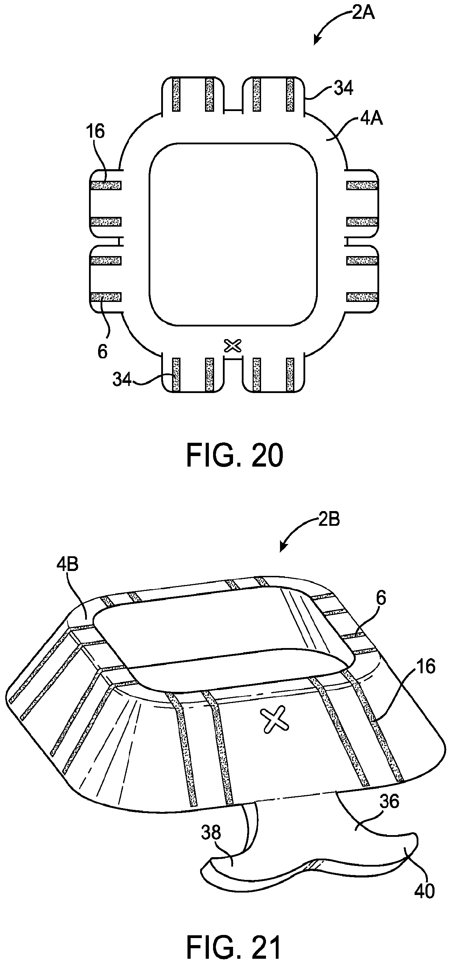

The main body 4 of the device 2 forms a closed loop having a central opening 26 with the cervix C positioned in the central opening 26. The main body 4 may be substantially circular or may have substantially straight sides 28 as shown in Fig. 21. Of course, the main body 4 may take any other shape such as oval, elliptical, square, or even hexagonal. Although the main body 4 extends completely around the cervix, the main body 4 may extend only partially around the cervix. To this end, the main body 4 may extend around at least 180 degrees, or at least 270 degrees, around the cervix relative to a cervical axis 30 (which is the same as the central axis CA of the device) and may be, for example, C-shaped, V-shaped or U-shaped. As used herein, the central opening 26 does not need to be completely surrounded by the main body 4 so long as the main body 4 extends partially around the cervix and opening 26 as described herein. Thus, a substantially C-shaped or U-shaped main body still will include a central opening 26 with each central opening 26 defining a central axis of the main body 4 CAB. The nerve stimulating elements 6 (such as the electrodes 16) are also spaced apart at least 120 degrees, or even 180 degrees, relative to the cervical axis CVA (or the central axis of the main body CAB described further below when the cervix is absent) so that the various nerve plexuses may be stimulated as described herein. Of course, the nerve stimulating element(s) 6 may be positioned only along a posterior or anterior half of the vaginal canal for targeted use as described herein without departing from numerous aspects of invention.

The device 2 of the present invention may be positioned partially or entirely within the vaginal fornices VF. The nerve stimulating element 6 (and in some embodiments all of the nerve stimulating elements 6) contacts the exposed surface of the vagina 10 proximal to the distal end 46 of the cervix.

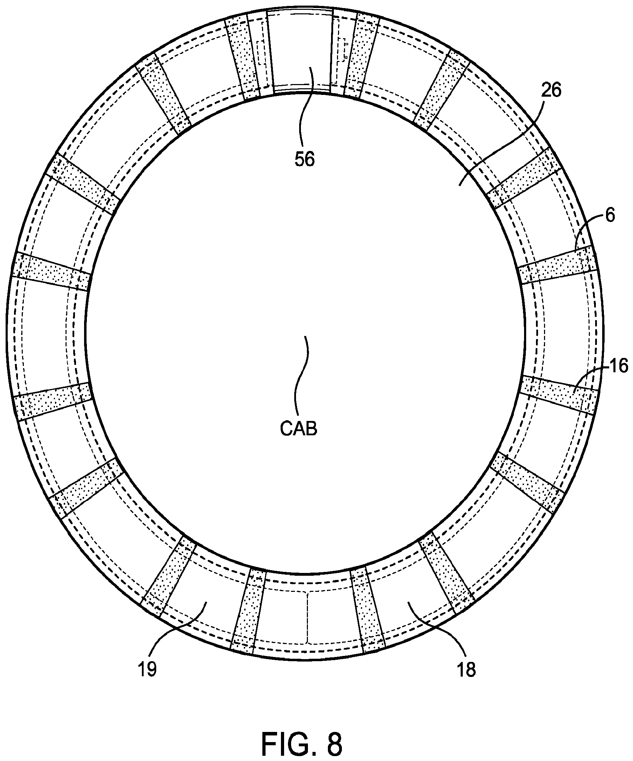

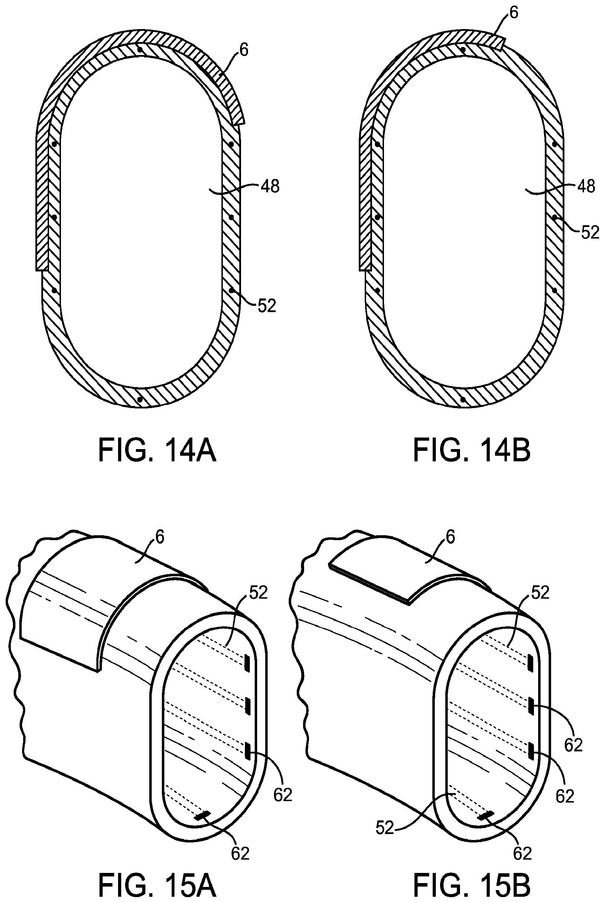

The main body 4 has an elongate cavity 48 (see Figs. 14A and 14B) in which the control system 18 and the battery 20 are positioned as shown in Fig. 8. The battery 20 and control system 18 are directly coupled together. The electrodes 16 are coupled to the control system 18 with a wire 52 (or other suitable conductive element) electrically coupled to each of the electrodes 16. The wires 52 extend through a sidewall 54 of the main body 4 and are directed toward a hub 56 which electrically couples each of the wires 52 to the control system 18. The hub 56 is positioned between a first connector 58 and a second connector 60. Half of the wires 52 are directed to each of the first and second connectors 58, 60 (eight wires 52 for each of the connectors 58, 60 in the preferred embodiment of Fig. 8). The wires 52 terminate at wire contacts 62 which are used to electrically couple the wires 52 to the hub 56 and, in turn, to the control system 18 as now described. Referring to Fig. 14A and 15A, the electrode 16 may wrap around from a radially outer surface 53 to a radially inner side 55. An alternative electrode 57 is shown in Fig. 14 B and 15B in which the electrode 57 does not wrap around to a radially inner side 59 thereby potentially minimizing stimulus transmitted through the cervix. All features, devices, embodiments and methods described herein may use either the electrode 16 or electrode 57.

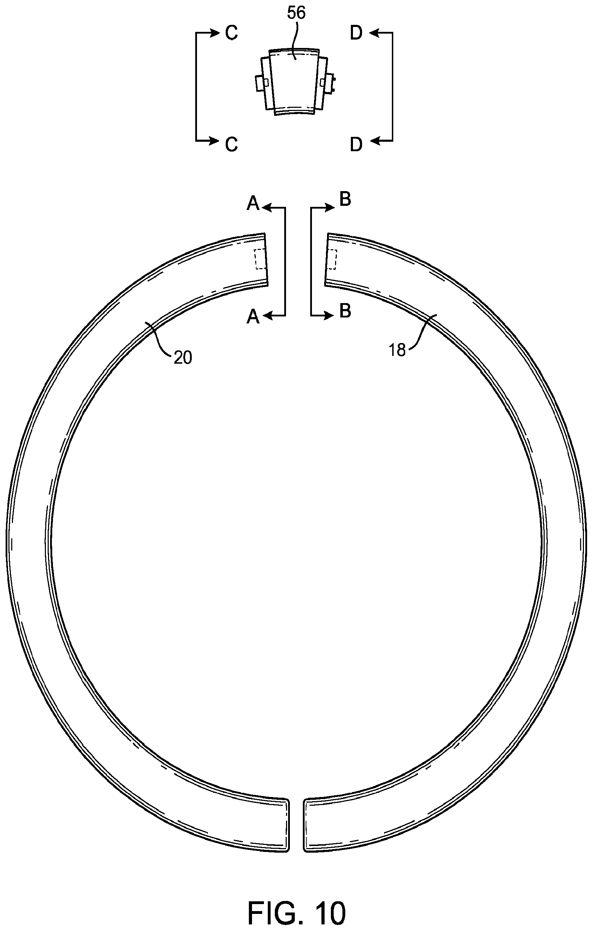

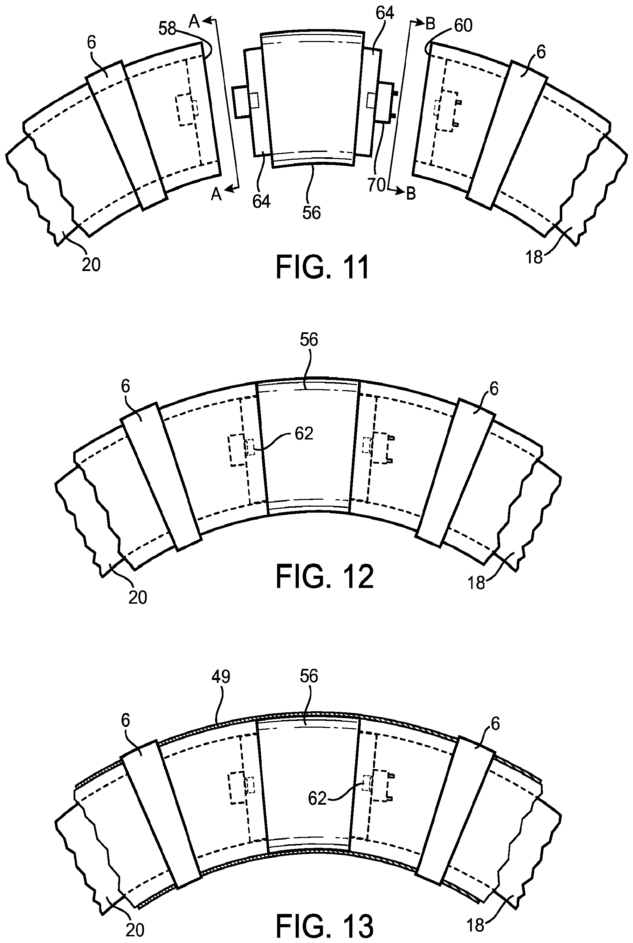

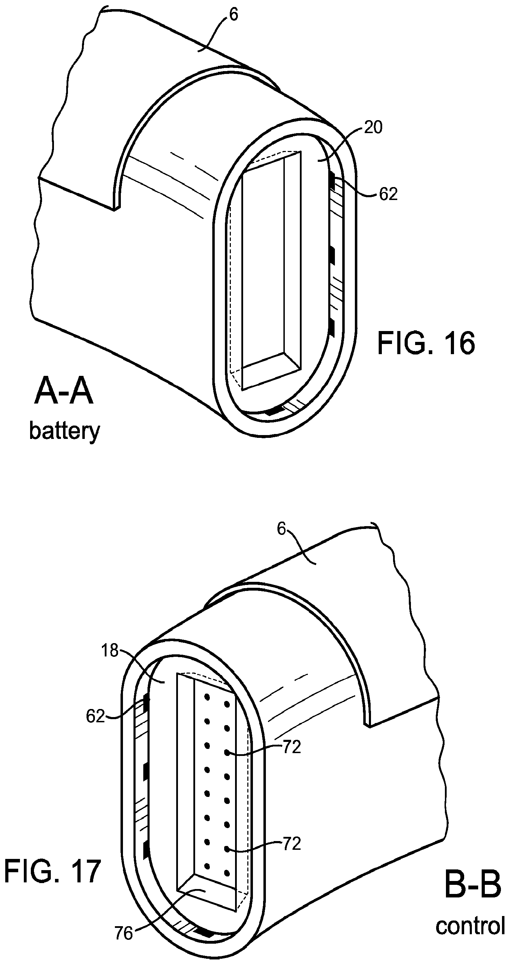

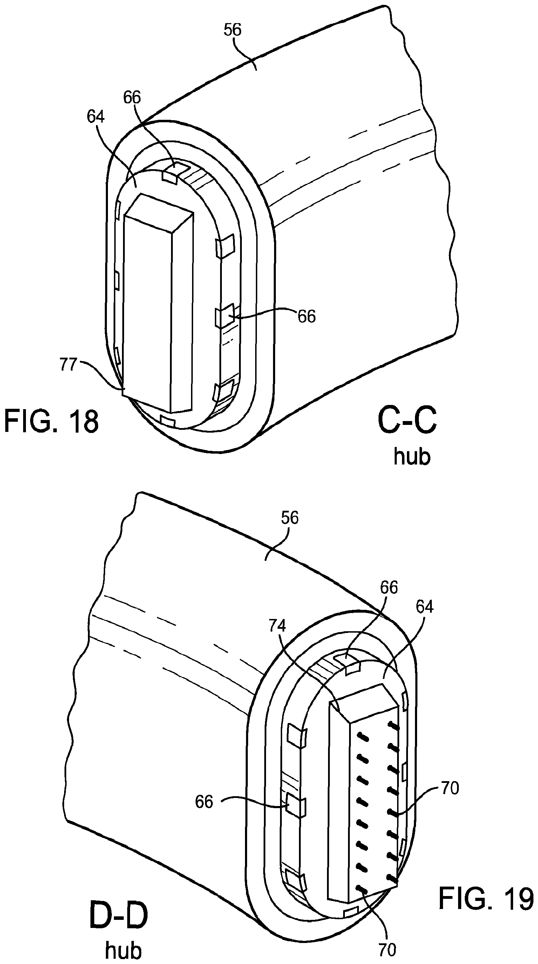

Referring to Figs. 10-19, the hub 56 has a protrusion 64 on each end that fits within the cavity 48 in the main body 4. The protrusion 64 on the battery 20 side abuts directly against the battery 20 (see Figs. 16 and 18) and the other protrusion 64 abuts against the control system 18 (see Figs. 17 and 19). The protrusion 64 includes hub contacts 66 on a radially outer wall that are aligned and electrically coupled to the wire contacts 62 at each of the first and second connectors 58, 60. Wires (not shown) leading from the hub contacts 66 are directed through the hub 56 to the control system 18. Each of the wires terminates at an electrical connector 70 (Fig. 19) that engages electrical connectors 72 on the control system 18 (Fig. 17). The electrical connectors 72 may be formed on a first extension 74 of the hub 56 that forms a snap fit connection with a recess 76 in the control system 18. A second extension 77 on the other side of the hub 56 may form a snap fit connection with the battery 20 (Fig. 18). Of course, the device 2 may prevent access to the battery 20 and control system 18 rather than providing the snap fit connection. For example, the entire device 2 may be encased in a polymer 49 to remove spaces and voids and to seal all connections to the hub 56 while leaving the electrodes 16 exposed as shown in Fig. 13.

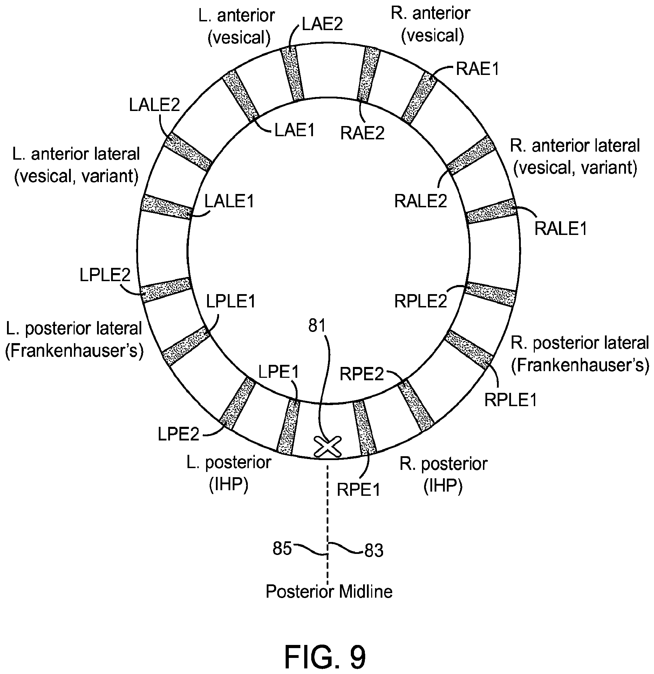

Referring now to Fig. 9, the electrodes 16 may be paired together to stimulate a specific nerve plexus, and even a specific side of the plexus, as now described. The device 2 includes a marker 81 to orient the device 2 along a midline 85 which generally corresponds with a midline 83 of the user. Adjacent the marker 81 is a left posterior electrode pair LPE1, LPE2 and a right posterior electrode pair RPE1, RPE2. The left and right posterior electrode pairs are positioned to lie adjacent to the left and right sides of the inferior hypogastric plexus. A left posterior lateral electrode pair LPLE1, LPLE2 and a right posterior lateral electrode pair RPLE1, RPLE2 are positioned to lie adjacent the left and right Frankenhauser's plexuses, respectively. Finally, the vesical plexus may be targeted on the left side with a left anterior lateral electrode pair LALE1, LALE2 and a left anterior electrode pair LAE1, LAE2 while the right side of the vesical plexus may be stimulated with a right anterior lateral electrode pair RALE1, RALE2 and a right anterior electrode pair RAE1, RAE2. Thus, the present invention may be useful in stimulating the same plexus from the right side and the left side simultaneously, independently and/or at different times. Of course, the user may only actuate the left or right side rather than alternating sides for stimulation if the therapy is tolerated better or more successful on one side or the other. The control system 18 may change the laterality periodically such as every few days.

Although the above description presents distinct electrode 16 pairings, it is understood that any of the electrodes 16 may be grouped together by the control system 18 for generating nerve stimulus. For example, the two electrodes 16 on opposite sides of the marker 81 may be used to stimulate the inferior hypogastric plexus along the midline 85 rather than preferentially on the left or right sides. In another example, the left hypogastric nerve (and the junction of this nerve and the IHP) may be stimulated using the LPE2 and the LPLE1 electrodes while the right hypogastric nerve (and the junction of this and the IHP) may be stimulated using the RPE2 and the RPLE1 electrodes. Although the independent stimulation of different regions of the same plexus has been described with respect to left and right sides of the midline of a particular target plexus, the stimulation may take place at any two different regions of the same plexus rather than simply left and right sides as described in further detail below.

Although only one electrode 16 is used on each side of the circuit described above, two or more electrodes 16 may be used to create either side of the electrical circuit rather than using only one for each side as described above. In another aspect of the present invention, the device 2 may include at least twelve electrodes 16 and in the preferred embodiment sixteen electrodes 16. The nerve stimulating element 6 may be formed by any two electrodes 16 (or groups of electrodes 16) and, thus, the device 2 could easily have at least twenty nerve stimulating elements formed by sixteen electrodes 16 by grouping electrodes together, and / skipping" over one or two electrodes 16 (or more) to form the electrode pair rather than using adjacent electrodes 16 to form the pair. An advantage of forming at least twenty different nerve stimulating elements 6 the device 2 may provide greater flexibility of treatment, reduce habituation and targeting different sides of the same plexus at different times as described herein. Skipping electrodes 16 to form a particular nerve stimulating element 6 will increase a spacing between the electrodes 16 (compared to adjacent pairs of electrodes 16) thereby providing the ability to potentially alter the depth of penetration of the stimulus. The control system 18 is configured to independently actuate each of the nerve stimulating elements 6. In this manner, the device 2 of the present invention may operate in at least twenty different modes with each mode being represented by a distinct nerve stimulating element 6 formed by a unique group of electrodes 16 in any manner described herein. Thus, the present invention not only is able to target numerous plexuses with a single device but each of these plexuses may be stimulated in a variety of independent modes. For example, the IHP may be stimulated with the four posterior electrodes 16 (LPE1, LPE2, RPE1, RPE2) in at least four modes (or stated another way by forming at least four different nerve stimulating elements) to stimulate the IHP. In fact, the four posterior electrodes 16 may form at least eight different modes (or stated another way by forming at least eight different nerve stimulating elements 6) to stimulate the IHP by simply grouping electrodes 16 in the manner described herein (adjacent pairs, skipping one, skipping two, grouping two or more electrodes on one side of the circuit or on both sides of the circuit). In this manner, different regions or portions of the same plexus may be stimulated. Of course, the regions stimulated will have overlapping portions but use of different electrode 16 configurations described herein will create different stimulus patterns and regions. Although the device 2 of the present invention may form numerous independent nerve stimulating elements 6, simultaneous actuation may produce fewer nerve stimulating elements 6 (such as eight when using sixteen electrodes 16), nevertheless, the device 2 still may form far more nerve stimulating elements 6 for independent actuation as described herein. The nerve stimulating elements 6 are also preferably formed by more than mere modification of power, frequency or another parameter for the same nerve stimulating element 6. As such, the nerve stimulating element 6 may each have a unique position in that the electrodes 6 are grouped, paired or otherwise organized in a unique manner for each of the nerve stimulating elements 6 formed. As such, each nerve stimulating element 6 formed has a unique position even if a grouping or pair share one or more electrodes 6 so long as the groups or pairings are not identical. Each unique position provides a different focus unlike a single nerve stimulating element that can only change power, frequency or some other characteristic while leaving the stimulation pattern substantially the same.

Although numerous nerve stimulating elements 6 may be formed with the electrodes 16 forming numerous different nerve stimulating elements, in some aspects of the invention the device 2 may have at least four, or at least eight, nerve stimulating elements 6 as described above. Each of the nerve stimulating elements 6 may be actuated independently (or simultaneously, of course) to stimulate different regions of tissue (although these regions may overlap). In another aspect of the invention, the nerve stimulating elements 6 are advantageously distributed around the main body 4 to independently stimulate the various target plexuses. To this end, the device 2 may include at least three nerve stimulating elements 6 which are angularly spaced at least 70 degrees from adjacent nerve stimulating elements relative to the cervical axis CVA or central axis of the main body 4 CAB. If more regions are targeted, the device 2 may include at least four nerve stimulating elements which are angularly spaced at least 50 degrees from adjacent nerve stimulating elements relative to the cervical axis CVA or central axis of the main body 4.

Although the nerve stimulating element 6 has been described with respect to pairs of electrodes 16, the nerve stimulating element 6 may be formed by a single element or even a single electrode 16 without departing from the scope of the invention. For example, a single piezoelectric element may be used to deliver ultrasound energy or the device 2 may include a single electrode 16 with the other electrode carried by another element (even an implantable element) without departing from the scope of the invention.

As mentioned above, many conventional devices introduced into the vagina suffer from the drawback that they often stimulate somatic nerves since these devices are typically positioned in the distal (lower) half of the vagina. These devices also often intend to stimulate pelvic muscles, which may lead to further disadvantages described herein. The present invention avoids these drawbacks by positioning the device 2 in the proximal half of the vagina and, in some embodiments, may have all of the nerve stimulating elements 6 (or electrodes 16) in the proximal half of the vagina. To this end, the present invention provides nerve stimulating elements 6 that are positioned close to the target plexuses; the vesical plexus, left and right Frankenhauser's plexuses, the inferior hypogastric plexus and the intersection of junction between the inferior hypogastric plexus and the left and right hypogastric nerves. These nerve plexuses travel close to the proximal end of the vagina as shown in Figs. 1-3 and typically have branches within 1-2 cm from the exposed surface ES of the vagina and, as such, the preferred embodiments are described with the nerve stimulating element 6 being no more than 3 cm from the target nerve plexus. Stated another way, the nerve stimulating element 6 may be positioned no more than 3 cm from the uterosacral ligaments which are adjacent the target nerve plexuses. Stated still another way, the nerve stimulating element 6 is positioned to stimulate the vesical plexus, Frankenhauser's plexus, or inferior hypogastric plexus without intervening nerves, and in particular without intervening somatic nerves. Stated yet another way, the nerve stimulating element 6 may be positioned to contact the exposed surface 10 of the vagina closer to the vesical plexus, left or right Frankenhauser's plexus, left and right hypogastric nerves or the inferior hypogastric plexus, than to the pelvic floor. Stated still another way, the nerve stimulating element 6 (and in some embodiments all of the nerve stimulating elements 6) is positioned to contact the exposed surface ES of the vaginal canal within 3 cm from a proximal end of the vagina or proximal to a distal end of the cervix C. The entire device 2 may be positioned proximal to a midpoint between the proximal and distal ends of the vagina. Stated another way, the entire device 2 may be positioned within 5 cm from the proximal end of the vagina. Stated still another way, the nerve stimulating elements 6 are all positioned proximal to a midpoint between the proximal and distal end of the vaginal canal.

The electrodes 16 are also oriented and organized so that they will form nerve stimulating elements 6 that will generally direct stimulus proximally. To this end, the electrodes 16, and the nerve stimulating elements 6, are organized so that a proximal surface 13 are at the same longitudinal position relative to the central axis of the body CAB and the cervical axis CA. Although the electrodes 16 and nerve stimulating elements are preferably oriented in this manner, they may be longitudinally separated without departing from numerous aspects of the present invention. For example, the electrodes 6 (and in one aspect all of the electrodes 16 or nerve stimulating elements 6) may be longitudinally spaced so that the proximal surface 13 of the electrodes 6 (and the nerve stimulating element 6 formed by the electrodes 16) relative to the central axis of the body CAB or the cervical axis CVA by no more than one cm.

Referring now to Fig. 20, another device 2A is shown including a main body 4A having one or more tabs 34 extending radially outward relative to a central opening 26A. The tabs 34 may help secure the device 2A in place and maintain the intended orientation of the device 2A once positioned. The electrodes 16 may be positioned on the tabs 34 so that the electrodes 16 are on the radially outer surface of the main body 4A.

Referring to Fig. 21, still another device 2B is shown which has an extension 36 extending radially outward from the main body 4. The extension 36 has a first curved tip 38 and a second curved tip 40 which help anchor the device 2 in position and help maintain the intended orientation. The main body 4B may also be flared outwardly which also may help retention. The main body 4B is flared outwardly to become larger as the body 4B extends distally in the vagina. It is understood that all of the devices 2, 2A, 2B described herein shall incorporate all methods of using the other devices 2, 2A, 2B and such use is expressly incorporated for each device described herein. Furthermore, all devices 2, 2A, 2B shall share the same inventive features described herein and such features are also expressly incorporated for all devices described herein. For example, the position and use of the electrodes 16 of device 2 shall be applicable to use of device 2A and device 2B.

The device may also be used to stimulate the vesical plexus together with either the Frankenhauser's plexus or with the inferior hypogastric plexus (or both). The vesical plexus may be stimulated independently or simultaneously with one or both of the other two plexuses. In another aspect, an anterior side may be stimulated simultaneously or independently with a posterior side.

As can be appreciated, the present invention provides the ability to target more than one plexus and, alternatively, the same plexus from a different position. For example, a plurality of nerve stimulating elements 6 are positioned to stimulate the same plexus for each of the plexuses described herein. Switching to different stimulating elements 6 may reduce habituation and/or provide other therapeutic benefits. Furthermore, the user may simply select, based on efficacy determined by the user, which of the areas is most beneficial for the particular treatment. Of course, a computer-controlled algorithm may also be used to target particular areas due to set parameters or from feedback data. The control system 18 may control the device 2 to stimulate a target plexus with a first nerve stimulating element 6 for a first period of time, then stimulate the target plexus with a second nerve stimulating element 6 for a second period of time and independent of the first nerve stimulating element 6. In this manner, habituation may be addressed without changing the target plexus but changing the position of stimulation by changing the nerve stimulating element. Of course, the target plexus(es) may also be changed from any one (or more) of the target plexuses described herein to another target plexus(es) to address habituation or as otherwise desired.

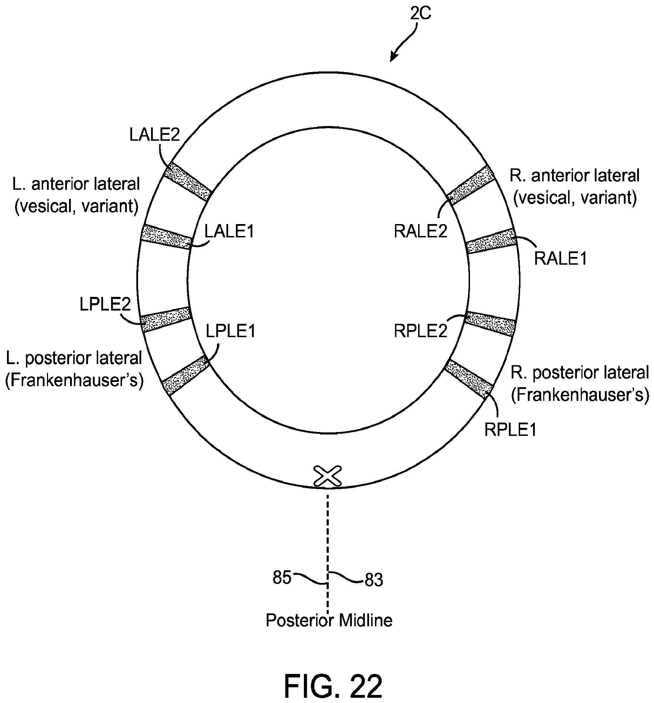

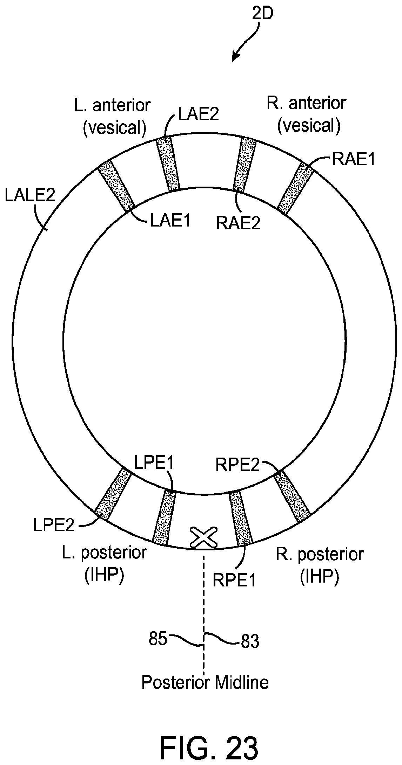

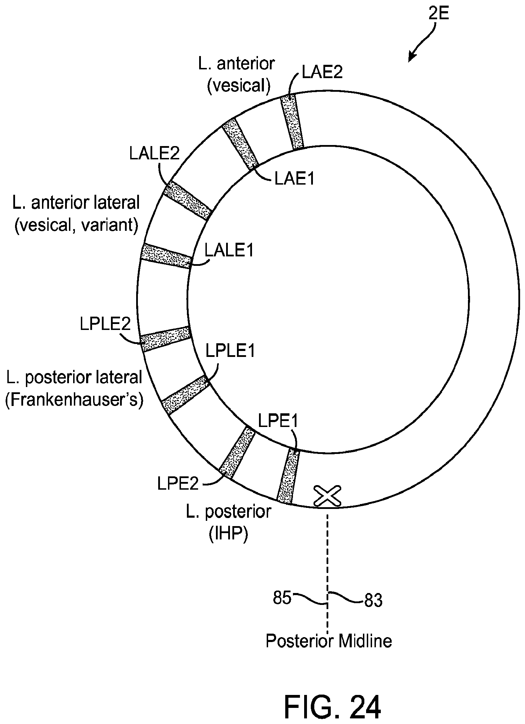

In one aspect, a plurality of devices 2C, 2D, 2E, such as at least three, having different patterns of stimulating elements 6 may be provided as shown in Figs. 22-24. Of course, any other suitable division of plexuses among the plurality of devices 2C, 2D, 2E may be provided. The user may switch between the devices 2C, 2D, 2E (or others with different patterns of elements) as desired. All aspects and methods of using the devices 2, 2A, 2B are incorporated here for use with the plurality of devices 2C, 2D, 2E as applicable.

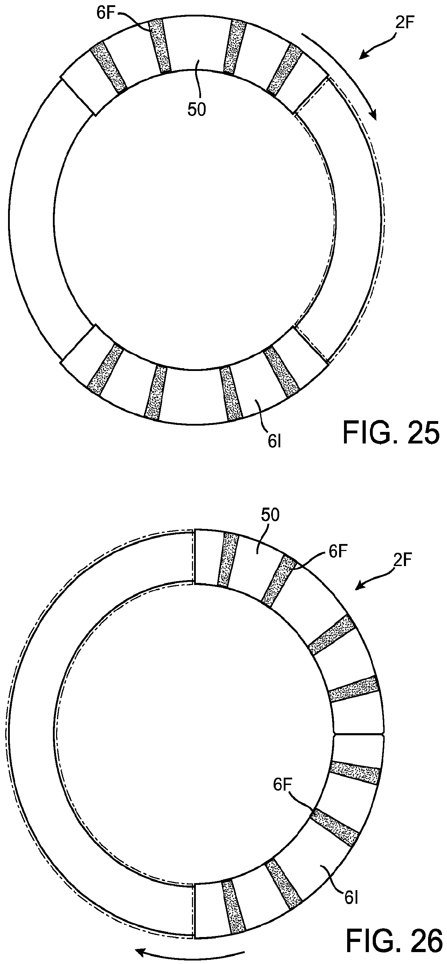

Referring to Figs. 25 and 26, the ability to select areas for stimulation may also be provided using device 2F having first and second movable elements 50,61. The first and second movable elements 50, 61 are coupled to a support body 4F which may extend in an arc of at least 270 degrees for positioning around the cervix. Each of the movable elements 50, 61 includes elements 6F for stimulating nerves and all aspects described herein of the devices 2, 2A, 2B, and 2C-2E are incorporated here. The first and second movable elements 50, 61 permit the user to move the elements 6F to desired positions for stimulating target plexuses. Fig. 25 shows the movable elements 50, 61 positioned to stimulate nerves when positioned on opposing sides of the cervix (any opposing regions). Fig. 26 shows the movable elements 50, 61 moved together to form a section, which permits stimulating half of the regions discussed herein and such uses are expressly incorporated here.

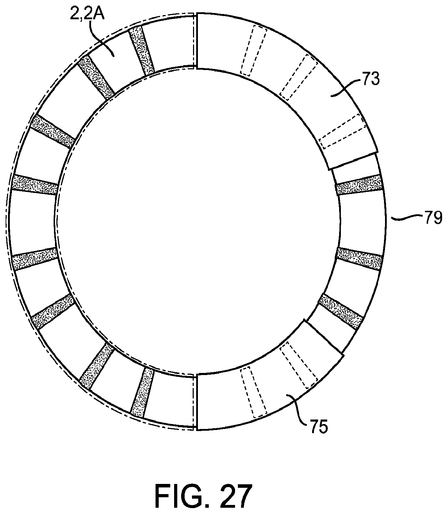

Referring to Fig. 27, a first cover 73 and a second cover 75 are provided to prevent or reduce stimulating some areas for use with any of the devices 2, 2A-2F described herein. The first and second covers 73, 75 are movable along the body to cover elements 6 as desired. The first and second covers may 73, 75 may also be spaced apart to form a window 79 for stimulating tissue within the window 79.

In still another aspect of the present invention, the device 2 is now referred to again with reference to Figs. 1-22. The device 2 itself may be used to orient the device 2 within the patient using the nerve stimulating elements 6 themselves. For example, when one of the nerve stimulating elements 6 emits energy, another of the nerve stimulating elements 6 may measure energy received from the emitting element 6. The control system 18 monitors the energy received at the other nerve stimulating element 6 and records data regarding the energy received. The control system 18 may then recognize the orientation of the device 2 relative to the cervix by recognizing a pattern in the energy received. In another aspect, the control system 18 may simply measure impedance between two or more nerve stimulating elements. The resulting impedance data may also be subsequently used to orient the device 2 with the control system 18 recognizing a pattern of data to orient the device 2. Stated another way, the control system 18 may be used to orient the device with at least one of the nerve stimulating elements emitting energy and at least one other of the nerve stimulating elements measuring the energy emitted. The control system 18 may save a pattern of a parameter and compare the pattern to current data to orient the device about the cervix. For example, RF energy or ultrasound energy may be emitted by one or more elements 6 and received at others with the control system 18 monitoring and storing the data. In another aspect, impedance between nerve stimulating elements 6 may be used with the control system 18 monitoring and storing the impedance and matching a pattern of saved impedance data to orient the device 2, 2A. The device 2 may then change the pattern of stimulation using the nerve stimulating elements 6 in accordance with the orientation determined by the control system 18. Of course, an angular shift of the device 2 around the cervix may orient different elements 6 adjacent different targets but, of course, such a shift and change of nerve stimulating elements 6 is clearly contemplated with the present invention and, as such, the particular elements 6 used to stimulate the target area (plexus) may change.

This ability to measure and compare parameters provides numerous additional benefits. The example of measuring impedance can, for example, provide data on blood and tissue. In general, the ability for electrodes to measure as well as deliver energy can provide other data on electrical properties of the tissue and fluids in the environment of the device. Both the ability to acquire measurements at different points in time, and the fact that the device 2 extends further in space than leads of implanted devices, allow for comparisons of parameters by the device. The ability of the device 2 to communicate with other medical and computing devices internal and external to the body extend all of these capabilities. The device 2 can incorporate any of a wide range of other sensors into the nerve stimulating elements or communicate with such sensors elsewhere on the device or on other devices internal or external to the body. Thus, a wide range of parameters can be sensed and monitored, diagnosed, and therapeutically treated. The data integration of such a device worn internally is novel in utilizing multiple sources and extending from sensing, through diagnosis, to therapy.

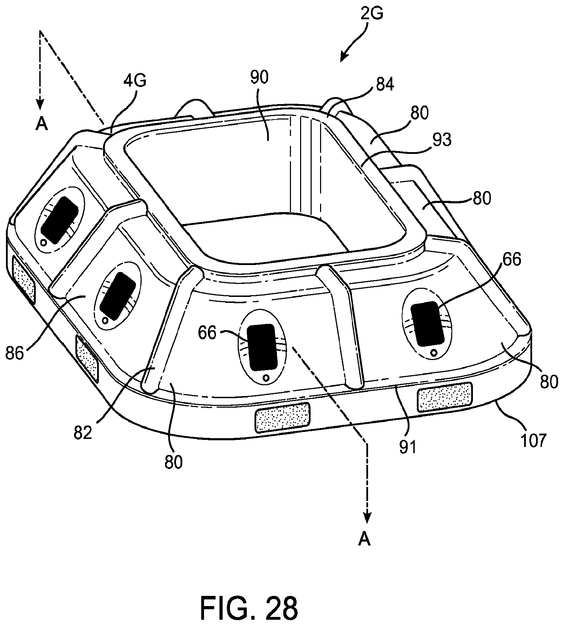

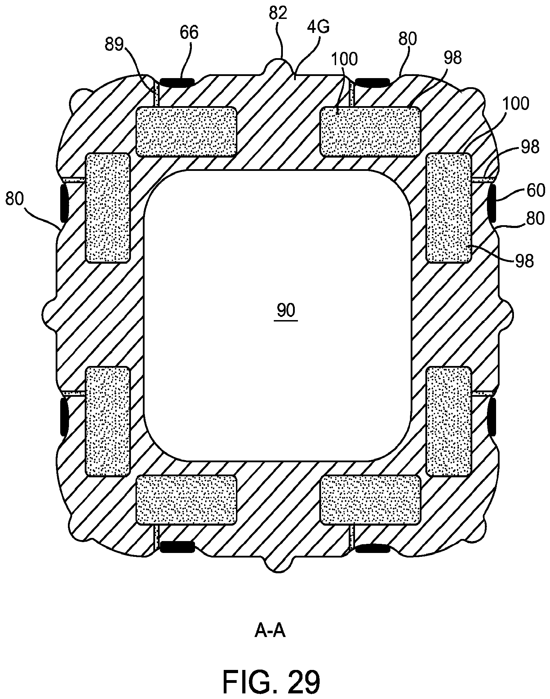

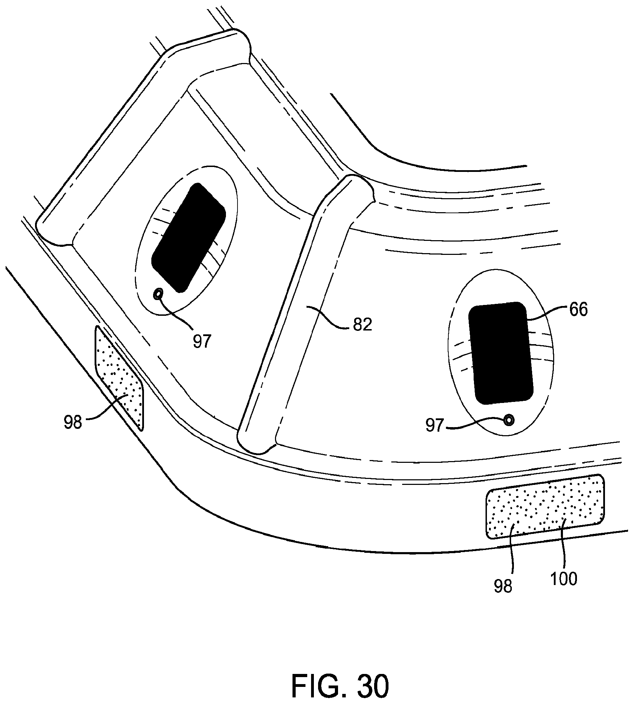

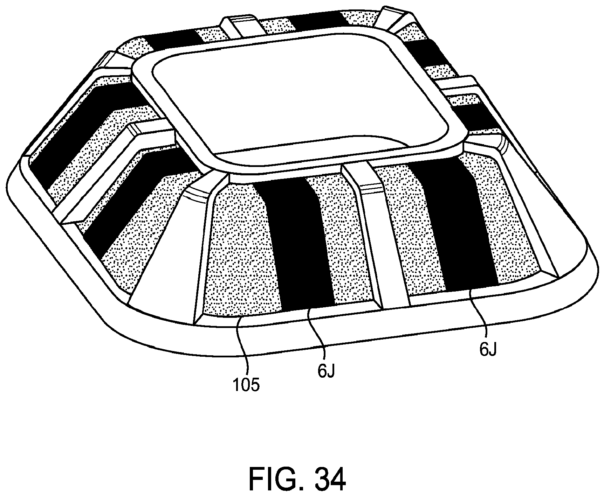

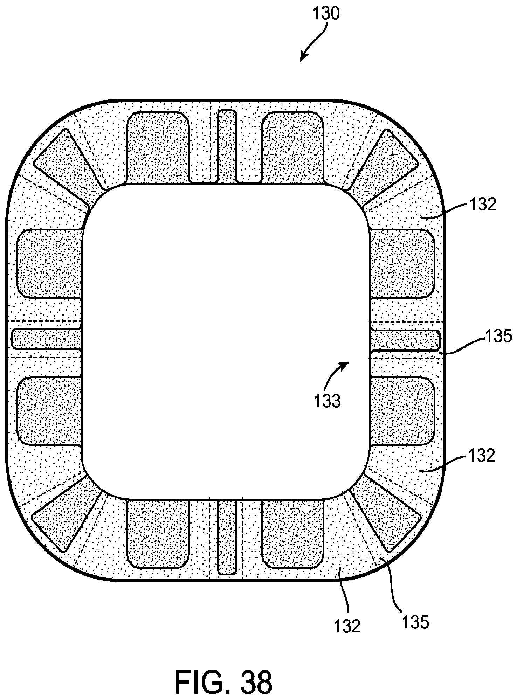

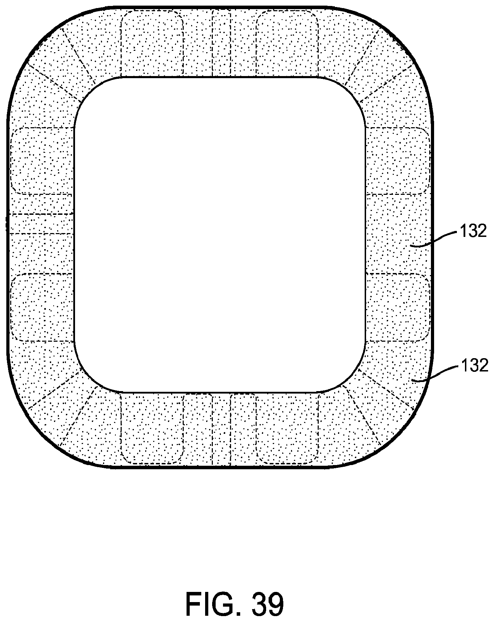

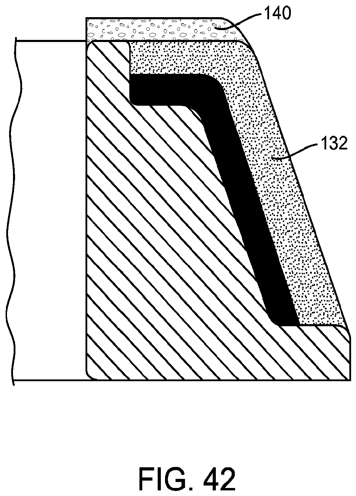

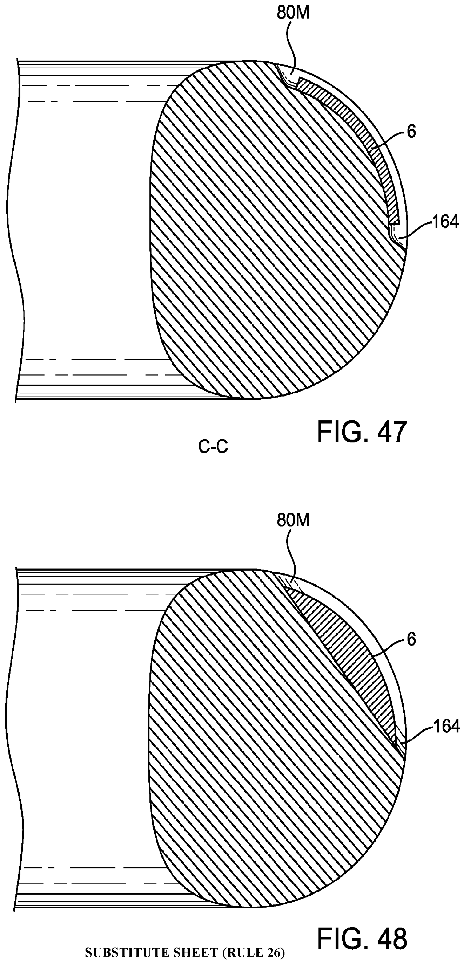

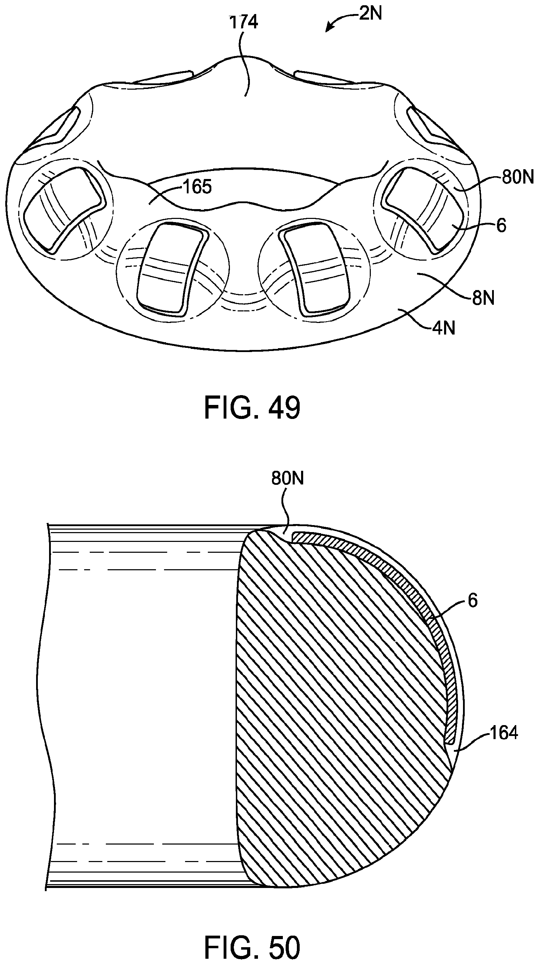

Referring to Figs. 28-51, various additional devices are shown. As with all other embodiments in this application, all methods of use and physical characteristics mentioned herein for one embodiment are equally applicable to all other embodiments. Thus, for the embodiments of Figs. 28-51, all methods of stimulating any of the target nerves or plexuses, or for any other therapeutic use mentioned herein, are incorporated here. For example, all devices described herein may include a first nerve stimulating element stimulating nerves adjacent the vagina, a second nerve stimulating element positioned to stimulate nerves on an opposing side of the cervix from the first nerve stimulating element when viewed along a cervical axis (independently or simultaneously and without moving the device). Similarly, all other applicable methods may be used specifically with every other embodiment. Furthermore, all physical characteristics, such as size, orientation, number and position of nerve stimulating elements, are also all incorporated here and for all embodiments described herein. Finally, although not specifically shown for each embodiment, each device includes the power supply, such as the battery, and the control system described herein and the characteristics of each are applicable to all embodiments.

Referring now to Figs. 28-31, another device 2G for stimulating nerves is shown wherein the same or similar reference numbers refer to the same or similar structure. As mentioned above, all features of corresponding structures and methods shall be incorporated here. For example, the nerve stimulating element 6G, although shaped uniquely compared to the other nerve stimulating elements, nevertheless may incorporate any of the features of the nerve stimulating elements described above such as the nerve stimulating element 6G may be an electrode 16G. The nerve stimulating elements 6G are positioned on an exterior surface 8G of a main body 4G. The main body 4G may form a closed ring 88 having a central opening 90 in which the cervix is positioned. The term "closed ring" or "closed loop" as used herein shall mean any closed shape regardless of shape and, as such, all of the closed shapes described herein shall constitute closed rings or closed loops.

The exterior surface 8G of the main body 4G forms a truncated pyramid having four sides 92 and rounded edges. At least one nerve stimulating element (or at least two) is positioned on each side 92. Alternatively, at least three of the four sides 92 may each have at least two nerve stimulating elements 6G.

The main body 4G includes a recess 80 in which the nerve stimulating element 6G is positioned. The main body 4G may include at least four recesses 80 and at least four nerve stimulating elements 6G each positioned in one of the recesses 80. The main body 4G may also include a non-conductive wall 82 extending between the recesses 80. The wall 82 may be electrically insulated (non-conductive) to isolate the nerve stimulating elements 6G from one another. The wall 82 may also help to contain a conductive material within the recess 80 as explained further below. The main body 4G may also have another wall 84 that extends around the opening 90. The wall 84 may also be a non-conductive material to help isolate the nerve stimulating elements 6G from one another (particularly when electrodes 16 are used) and to contain a conductive material. The wall 84 is positioned between the nerve stimulating elements 6G and a radially inner edge 93 (smaller end) of a central opening 95 in the main body 4G. Another wall 91 is positioned at a radially outer edge 107 (larger end of the opening) of the central opening 90.

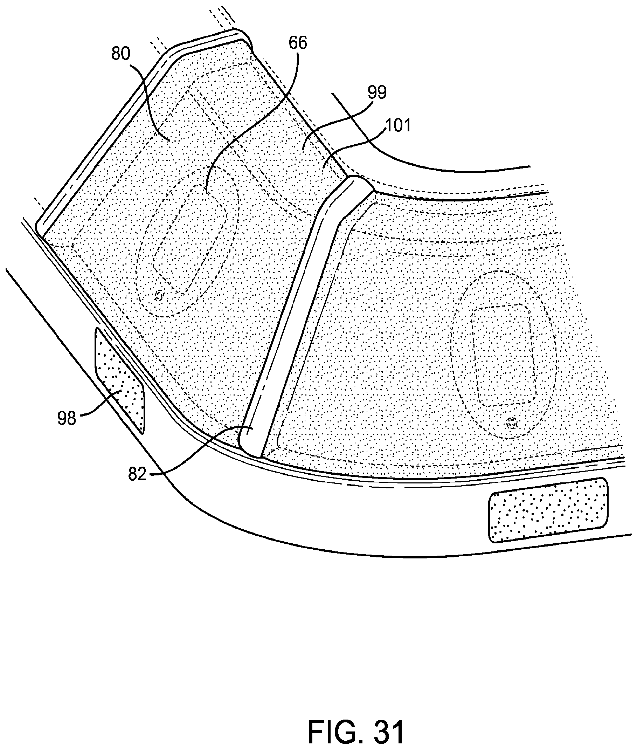

The main body 4G may also include a reservoir 94 which contains a flowable substance 96 such as saline or a hydrogel. The reservoir 94 has a lumen 89 fluidly coupled to the recess 80 and a permeable and/or porous structure 98 (depending on application) such as an open cell foam exposed and positioned in the recess 80. The structure 98 has pores 100 that hold the flowable substance 96 which may be electrically conductive (such as saline or a hydrogel). The flowable substance 96 may continue to weep from the structure 98, depending on the physical characteristics of the structure 98, lumen 89 and flowable substance 96, to position the flowable substance 96 in the recess 80 adjacent the nerve stimulating element 6G. The structure 98 may automatically draw the flowable substance 96 from the reservoir 94 thereby automatically replenishing the flowable substance 96 as necessary. Of course, the flowable substance 96 may also be forced from the reservoir 94 without departing from the scope of the invention. The flowable substance may be a conductive substance to enhance electrical contact between the nerve stimulating element 6G and tissue. A fill hole 97 may be used to fill the reservoir 94 as necessary. The reservoirs 94 may each deliver to a single recess 80 but may, of course, deliver to multiple recesses 80 without departing from the scope of the invention. Referring to Fig. 31, the flowable substance is shown filling the recess 80. The recess 80 may also be filled with a permeable material 99 which holds the flowable substance 96 in pores 100 therein as describe in connection with other embodiments described herein.

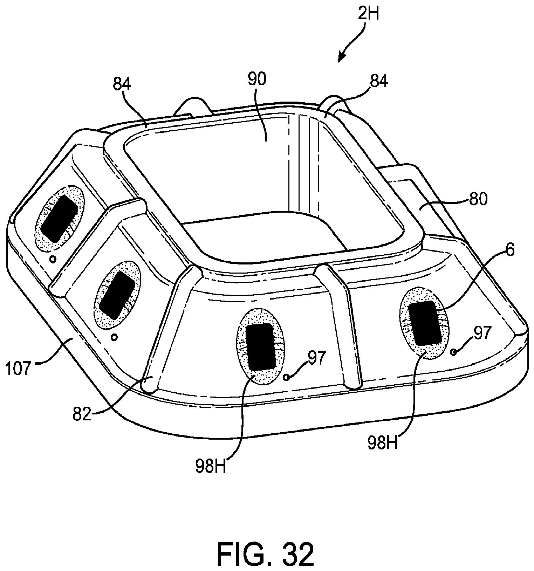

Referring to Fig. 32, another device 2H for stimulating nerves is shown wherein the same or similar reference numbers refer to the same or similar structure. The device 2H includes a permeable and porous structure 98H surrounding the nerve stimulating element 6H. In this manner, the flowable substance 96 is delivered directly to the area around the nerve stimulating element 6H. The flowable substance 96 weeps from the permeable structure 98 and is contained within the recess 80 with the walls bounding the recess 80 helping to contain the substance 96. The walls also provide a non-conductive barrier to reduce the likelihood of a short circuit between adjacent elements 6 (particularly when using electrodes).

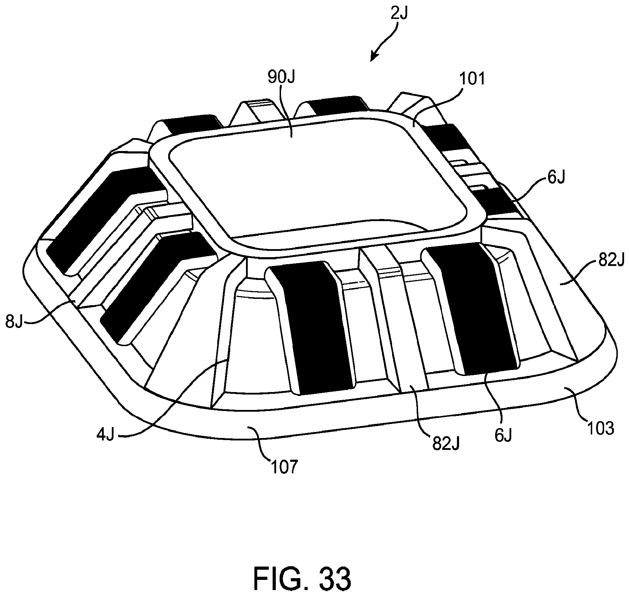

Referring to Figs. 33-35, another device 2J for stimulating nerves is shown wherein the same or similar reference numbers refer to the same or similar structure. The device 2J has nerve stimulating elements 6J on an exterior surface 8J of a main body 4J. The main body 4J may have the shape of a truncated four-sided pyramid and may include the features associated with this shape described above.

The main body 4J includes a wall 82J extending between recesses 80J. The wall 82J may be electrically insulated (non-conductive) to isolate the nerve stimulating elements 6J from one another. The wall 82J may also help to contain a conductive material 105 within the recess 80J as explained further below. The main body 4J may also have another wall 84J that extends around a smaller end 101 of central opening 90J. The wall 84J may also be a nonconductive material to help isolate the nerve stimulating elements 6J from one another (particularly when electrodes 16 are used) and to contain a conductive material. A lip 103 forms another wall 107 that extends around the larger end (radially outer side) of the central opening 90J thereby surrounding each recess 80J with walls 82J, 84J and wall 107. A conductive material 105 is positioned in the recess. The material 105 may be a soft, pliable, electrically conductive structure such as any of those described herein including a porous material or substance (using saline or a gel such as a hydrogel). In this manner, the conductive material 105 may conform somewhat to the shape of the tissue to enhance contact with tissue and electrical conduction.

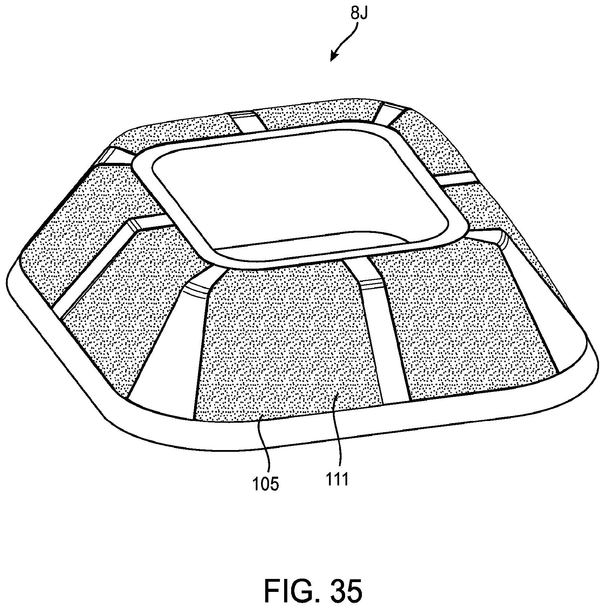

Referring to Fig. 35, the nerve stimulating element 6J may be covered by a pliable, electrically conductive material 109. The conductive material 109 may also be pliable so that the conductive material 109 also conforms somewhat to the shape of the tissue. The conductive material 109 may be a permeable open cell foam element I11 with a conductive fluid, such as saline, contained in the pores of the element I11.

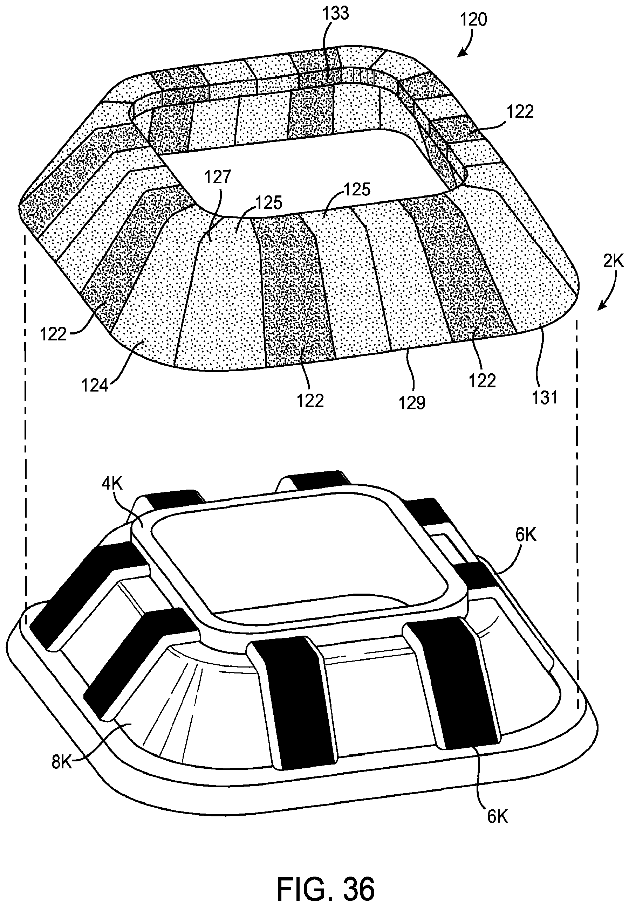

Referring to Fig. 36, still another nerve stimulating device 2K is shown wherein the same or similar reference numbers refer to the same or similar structure. Nerve stimulating elements 6K are positioned on a main body 4K having an exterior surface 8K having a shape similar to a truncated four-sided pyramid and all characteristics of this shape described herein are incorporated here. The device 2K includes an insert 120 which may be a separate device that is attached to the main body 4K by the user. Alternatively, the insert 120 may be integrated into the device 2K without departing from the scope of the invention.

The insert 120 has conductive elements 122 positioned over the nerve stimulating element 6K which may be pliable to conform to tissue and thereby enhance electrical conduction. The plurality of conductive elements 122 are coupled to and separated by nonconductive elements 124 which may also be pliable. The electrically conductive element 122 may be a gel, such as a hydrogel, or a porous material 126 having a flowable material, such as saline, contained in the pores of the porous material 126. To this end, the term electrically conductive shall include porous materials that are not electrically conductive but can conduct electricity when the pores are filled with an electrically conductive material. The non-conductive element 124 may also be a thin layer of material such as a closed cell structure made of a nonconductive material. Alternatively, the non-conductive element 125 may simply be a pliable layer 127 of non-conductive material such as silicone. The pliable nature of the layer 127 may help to conform to tissue to create a barrier to electrical conduction between adjacent nerve stimulating elements 6k. The insert may include a main body 129 that may simply be an elastic band 131 at a radially outer (larger end) of a central opening 133. The band 131 may be coupled to one or both of the non-conductive elements 125 and/or conductive elements 122.

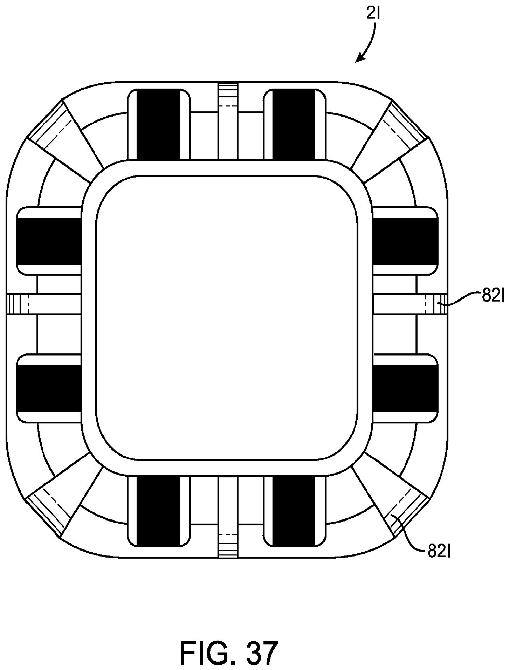

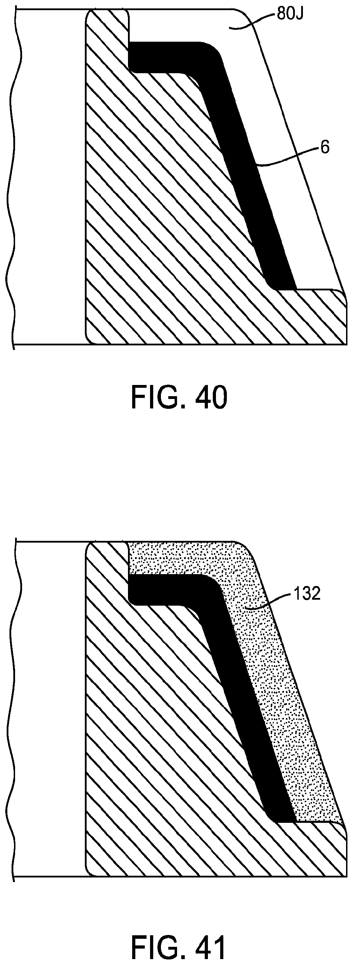

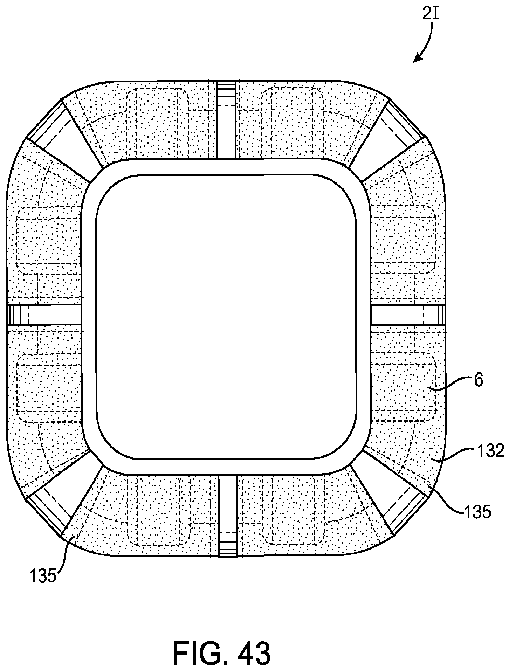

Referring to Figs. 37-43, another device 21 is shown for use with an insert 130 wherein the same or similar reference numbers refer to the same or similar structure. The device 21 is substantially the same as the device 2K and all aspects of device 2K are incorporated here. The insert 130 has conductive elements 132 mounted to a main body 137, which may form a closed loop 145, at spaced apart locations. The conductive elements 132 may be any of those described herein including the pliable structures described above. The main body 137 includes non-conductive elements 135, such as a thin layer, positioned between the conductive elements so that the conductive elements 132 are coupled to and separated by non-conductive elements 135. The main body 137 may include a dissolvable portion 142 that dissolves within the body under normal conditions (vagina in this application). Referring to Figs. 41 and 43, the dissolvable portion 142 may include the non-conductive elements 135 thereby leaving only the electrically conductive elements 132.

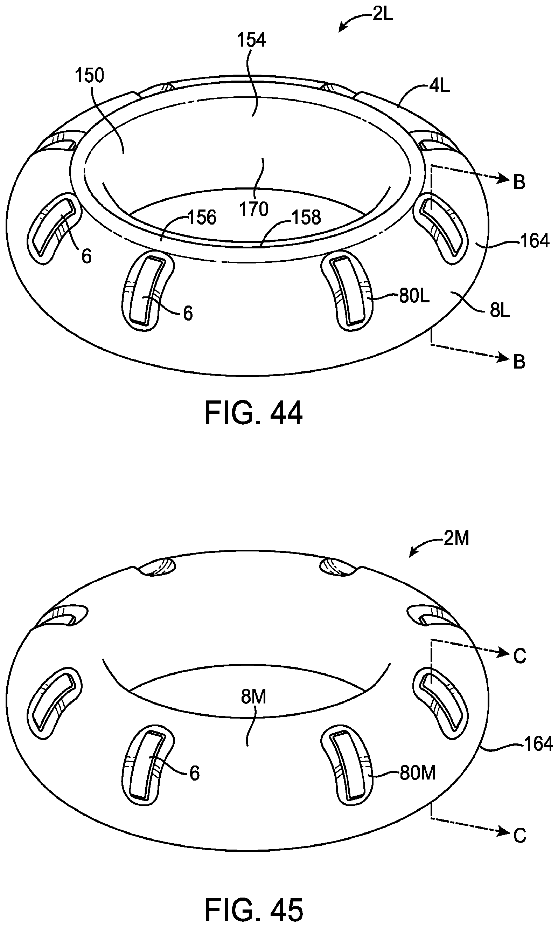

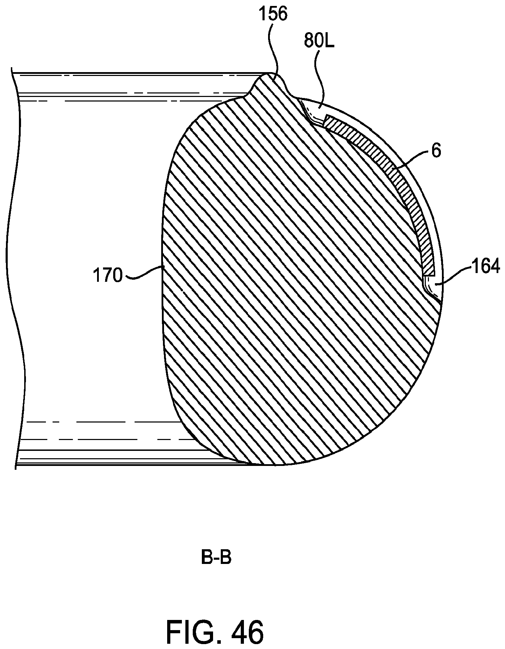

Referring to Figs. 44-51, a device 2L, a device 2M and a device 2N are shown having bodies 4L, 4M, 4N with an exterior surface 8L, 8M, 8N that each form a torus. As used herein "torus" shall apply to shapes that are torus-like or generally torus-shaped. The devices 2L, 2M and 2N also form a closed ring 150 like other embodiments described above. The nerve stimulating elements 6 are positioned in recesses 80L, 80M, 80N as shown in the cross-sectional view of Figs. 46, 47, 48 and 50. The main body 2L of the device 2L has a central opening 154. The main body 4L has a non-conductive wall 156 which may be a closed loop 158 adjacent the opening 154 and positioned between the nerve stimulating element 6 and a radially inner side 170 of the torus. The recesses 80L, 80M, 80N and nerve stimulating elements 6 are positioned predominantly on a radially outer side 164 of the torus. The device 2N includes recesses 80N which form a scalloped shaped wall 165 extending around the central opening 174 and positioned between the nerve stimulating element 6N and the radially inner side 170 of the torus. As shown in the various cross-sectional views, the exterior surface 8L, 8M, 8N at the radially inner side 170 of the torus may be somewhat flattened.

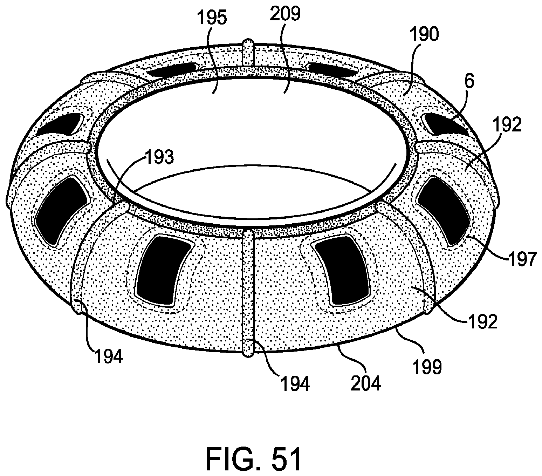

Referring to Fig. 51, another insert 190 is shown which may be applied to any of the devices 2L, 2M, and 2N. The insert 190 may be integrated with the devices 2L, 2M, 2N or may be a separate, disposable device that is applied by the user. The insert 190 has conductive elements 192 separated by non-conductive elements 194 similar to the other inserts described above and all discussion above is equally applicable and incorporated here concerning the conductive element 192 and non-conductive element 194. The non-conductive element 192 may, for example, be a layer 193 of non-conductive material such as silicone. The insert 190 may include a body 204 which may simply be an elastic band 199 extending around one end to secure the insert 190 onto the devices 2L, 2M, 2N. The body 190 may form a closed loop 195 (as broadly described herein) with the elastic band 199 circumscribing the closed loop 195 around the central opening 209. The non-conductive element 194 may also form a raised wall 211 relative to the conductive element 192. The raised wall 211 may also be a thin layer of silicone. The insert 190 may include openings 197 through which the nerve stimulating element 6 is exposed. The openings 197 are positioned to expose the nerve stimulating elements 6 with at least one nerve stimulating element 6 in each opening 197. The term "insert" as used herein is used to describe a disposable device used with a reusable part although the insert may, in fact, not be "inserted" into any part of the device and, as shown, may in fact simply surround the other reusable device. Of course, at times the insert is introduced into another object (such as a recess) yet such interaction is not required. The term "system" as used herein may refer to an integrated, single piece device and does not imply or require multiple parts (other than those integrated into a system). Of course, the system may still be provided in separate parts, such as separate controller, control system or battery external to the vagina, without departing from the invention as well since a system may still include separate components coupled together wirelessly or with wires.

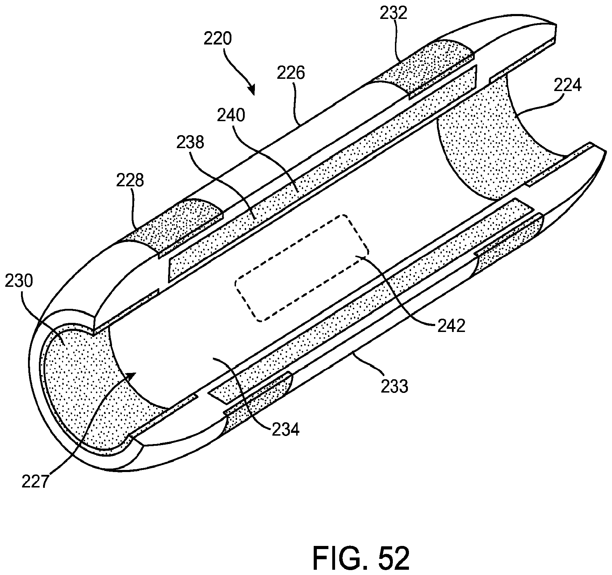

Referring now to Figure 52, an implant 220 in accordance with the present invention is shown. The implant 220 is used to stimulate nerves independently, in conjunction with other implants 220 or in conjunction with any of the devices 2, 2A-2N. The implant 220 is surgically placed and secured adjacent to the uterosacral ligament so that a first nerve stimulating element 224 (coupled to an implant body 226) is positioned in contact with the uterosacral ligament. The implant body 226 may be secured directly to the uterosacral ligament with the uterosacral ligament positioned in a throughhole 227 extending through the implant body 226. Alternatively, the implant 220 may be secured to another structure with the nerve stimulating element 224 positioned in contact with the uterosacral ligament. A second nerve stimulating element 228 may be positioned on an outer surface 233 of the implant body 226 so that it is in contact with the peritoneum after placement. A third nerve stimulating element 230 (also coupled to the implant body 226) may also be provided and positioned in contact with the uterosacral ligament at an opposite end of the throughhole 227 from the first nerve stimulating element 224. A fourth nerve stimulating element 232 may also be in contact with the peritoneum and spaced apart from the second nerve stimulating element 228. For the purpose of defining the invention and in particular the claims, the numerical nomenclature associated with the nerve stimulating elements and other structures, such as the "fifth nerve stimulating element" shall not carry numerical significance in that reciting the "fifth nerve stimulating element" does not necessitate four additional nerve stimulating elements and the numerical significance shall not be taken into account. Thus, the "fifth nerve stimulating element" may, in accordance with the claims, be the second recited nerve stimulating element with only two recited. Use of the "fifth nerve stimulating element" may, in fact, be the first (and possibly only) nerve stimulating element for a particular implant but is used since another implant recites (at times in subsequent dependent claims) the second, third and fourth nerve stimulating elements. All aspects of the nerve stimulating elements described herein are applicable to the nerve stimulating elements of the implant 220 and all features and characteristics are incorporated here for the nerve stimulating elements of the implant 220.

The uterosacral ligament passes through a slot 234 when introduced into the throughhole 227. The implant body 226 is movable from an open position, which permits the uterosacral ligament to enter the throughhole 227 to a locked position which prevents the uterosacral ligament from moving out of the throughhole 227. The locked position may also be provided in any suitable manner such as with a separate structure including as a suture, clasp, cinch, clamp, or a sliding door or gate over the slot 234 without departing from the scope of the invention.

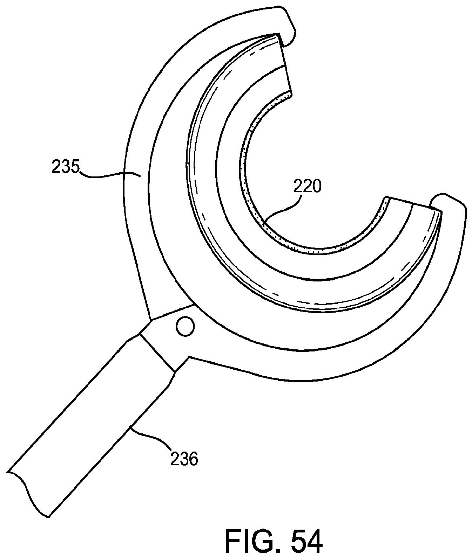

Referring to Figure 54, the implant body 226 may also be held in the open position with a delivery tool 236. The delivery tool 236 has a movable jaw 235 so that the delivery tool 236 can hold the implant 220 in the open position of Fig. 54 and release the implant 220 when desired. The implant body 226 is introduced into the abdomen in the open position and released when the uterosacral ligament is positioned in the throughhole 227. The implant 220 may naturally move to the locked position with the delivery tool 236 holding the implant 220 in the open position. Alternatively, the implant body 226 may be naturally biased to the open position and closed by the delivery tool 236 to clamp and secure the implant 220 to the ligament. Of course, the clamping force must be applied in a controlled manner to prevent excessive pressure on the uterosacral ligament while still adequately securing the implant 220. The implant 220 may include other securing features, such as one or more eyelets to receive suture passed through the uterosacral ligament, without departing from the scope of the invention.

The implant 220 also includes a power source 238, such as a battery 240, coupled to the implant body 226. A control system 242 (positioned within the implant body 226) is coupled to the power source 238 and to the nerve stimulating elements in a conventional manner. The battery 240 may be rechargeable (not shown) without departing from the invention and may be charged with a charging device positioned in the vagina in the same position as the device 2 as described in greater detail below.

As mentioned above, the first and third nerve stimulating elements 224, 230 are in contact with the uterosacral ligament which itself is physically connected through various tissue paths to the target plexuses as described herein. Thus, the uterosacral ligament is used to transmit nerve stimuli to the target plexuses and nerves and the anatomic position of the uterosacral ligaments relative to the target areas is advantageous as described above. The nerve stimulating elements on the implant 220 may also be useful for stimulating other nerve plexuses such as the superior hypogastric plexus (SHP) which would be difficult to stimulate using the device 2. The nerve stimulating elements on the implant 220 may also be suitable for delivering stimulation to the middle rectal plexus to treat fecal urge, rectal conditions, and/or incontinence.

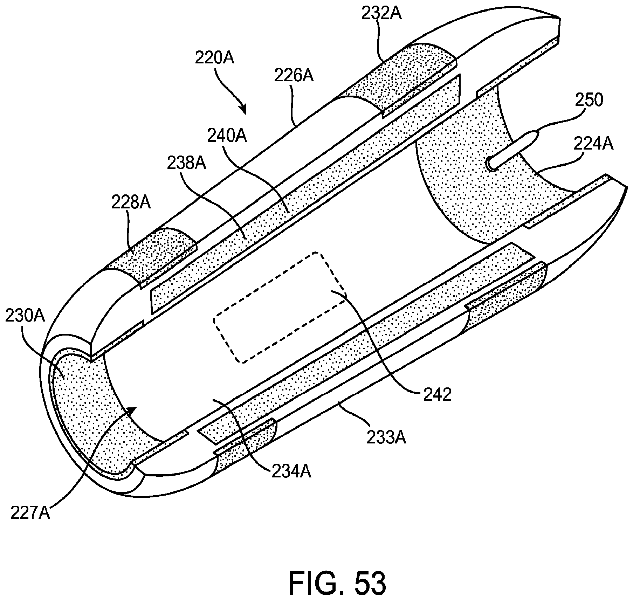

Referring to Figure 53, another implant 220A is shown wherein the same or similar reference numbers refer to the same or similar structure. The implant has a throughhole 227 A, a control system 242, and first, second, third and fourth nerve stimulating elements 224A, 228A, 230A, 232A. The throughhole 227 A is tapered to accommodate a tapered geometry of the ligament. When sizing the implant 220A, the size may be taken at two positions so that a tapered geometry may also be considered when selecting the appropriate size of implant 220A. The first nerve stimulating element 224A has a piercing element 250 configured to extend into and pierce the uterosacral ligament when securing the first nerve stimulating element 224 to the uterosacral ligament. To this end, the piercing element 250, may be a barb, needle, spike or pin extending into the throughhole 227. When the implant 220A is placed around the ligament the piercing element 250 automatically extends into and pierces the ligament. The piercing element 250 may help prevent migration (slipping) of the implant 220A along the ligament and also may improve stimulation by improving contact between the first nerve stimulating element 224A and the uterosacral ligament and associated autonomic nerves. The piercing element 250 is preferably made of a conductive material so that the piercing element 250 forms part of the first nerve stimulating element 224. It is understood that whenever implant 220 is described that implant 220A may be substituted with all features and methods incorporated.

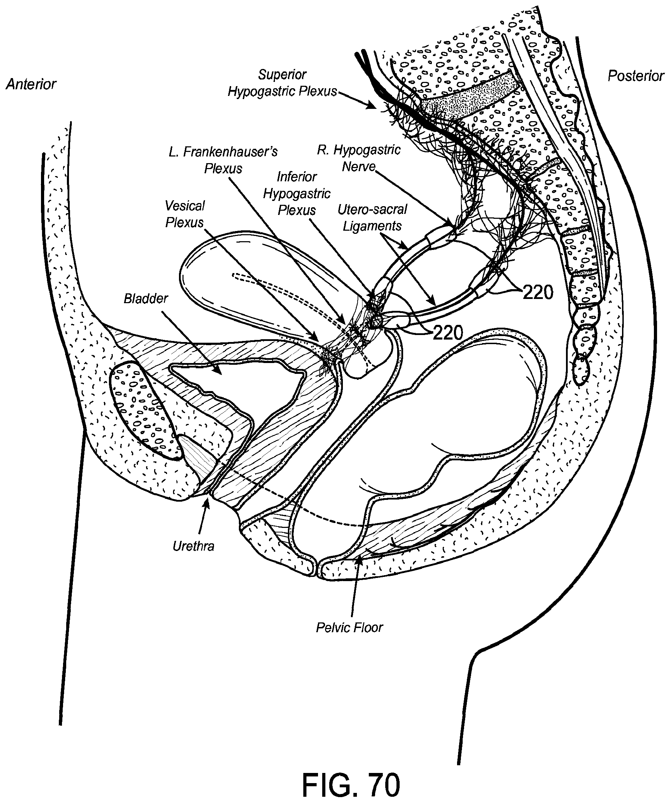

The implant 220 may also be small enough so that two implants 220 can be positioned adjacent (and even secured) to the same uterosacral ligament. If the implants 220 are separated with one positioned anteriorly on the uterosacral ligament and the other positioned posteriorly (as shown in Figure 70) it is possible to primarily stimulate the IHP with one implant and the SHP with the other.

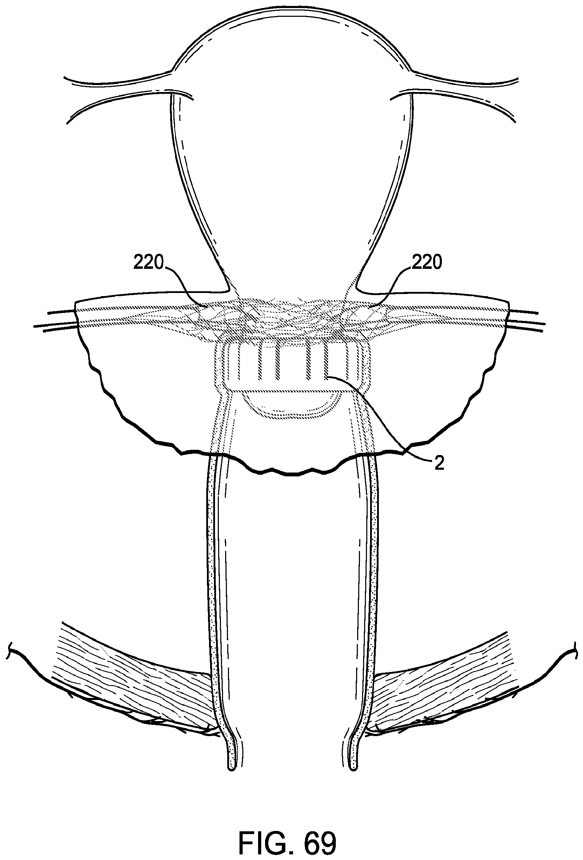

The implant 220 may be used by itself, together with additional implants 220, 220A or in conjunction with any of the devices 2, 2A-2N positioned in the vagina described herein and shown in Figure 69. It is understood that when device 2 is described for the combination with the implant 220 that any of the other devices 2A-2N may be used as a substitute and those features and methods of use, including all claims, are expressly incorporated. Furthermore, all aspects of the devices 2, 2A-2N positioned in the vagina and described herein may be used with the implant 220 of the present invention for stimulating nerves and nerve plexuses and all aspects, uses and characteristics of the nerve stimulating elements and methods for devices 2, 2A-2N are specifically incorporated here for combining with the implant 220 of the present invention.

The implant 220 and the devices 2, 2A-2N may work together to stimulate nerves to modulate the signal of the autonomic plexuses surrounding the cervix and uppermost aspect of the vagina, namely, the inferior hypogastric plexus (IHP), Frankenhauser's plexus (L and R) and the vesical plexuses (L and R). In this manner, the stimulation may alter systemic sympathetic discharge throughout the sympathetic chain. In a specific aspect, the device 2 may have two nerve stimulating elements 6. The two nerve stimulating elements 6 on the device 2 and the nerve stimulating element(s) on the implant 220 together stimulate at least three nerve plexuses from the following list; SHP, IHP, L vesical, R vesical, L Frankenhauser's, and R Frankenhauser's. For example, the implant 220 may stimulate the IHP (or the SHP) while the device 2 has at least one of the nerve stimulating elements 6 stimulating a side of the Frankenhauser's plexus (L or R) and another nerve stimulating element 6 stimulating the other side of the Frankenhauser's plexus relative to the midline 83 (see Fig. 22). Alternatively, the nerve stimulating elements 6 may be positioned with one nerve stimulating element 6 positioned on a side of the vesical plexus and another nerve stimulating element 6 positioned on the other side of the vesical plexus relative to the midline 83. The "other side" of a plexus as used herein shall mean any position on the opposing side of the midline to the other nerve stimulating element and, thus, the nerve stimulating elements positioned on "the other side" do not have to be 180 degrees from one another.

The device 2 may also communicate with the implant 220 to coordinate stimulation. For example, various therapies may require simultaneous stimulation while others require independent stimulation. The device 2 and the implant 220 may communicate by simply transmitting and receiving electrical signals through tissue using the nerve stimulating element 6 of the device 2 and the nerve stimulating element of the implant 220. For the purpose of the present invention, "communication" shall require either transmission or receipt of signals or information but does not require both. The controller 24 of the device 2 may be used to communicate with the control system 18 of the device 2 to control, for example, a duration of stimulation and an intensity of stimulation. The controller 24 may also be configured to communicate stimulation instructions for the control system 242 of the implant 220 to the device 2 which then communicates the instructions to the implant 220. Of course, the controller 24 may be configured to communicate instructions directly to the control system of the implant 220 as well. For example, the controller 24 (and/or the implant 220) may generate radiofrequency signals to communicate with one another in a conventional manner.

More than one implant 220 may be delivered and secured to the same uterosacral ligament. Each implant 220 may include all features related to the implants of the present invention. When more than one implant 220 is used, the control systems 242 of the implants 220 may also communicate with one another to coordinate stimulation (such as simultaneous stimulation or independent stimulation). The implants 220 may communicate using electrical signals transmitted to and from the nerve stimulating elements and transmitted through tissue. Alternatively, the nerve stimulating elements (or a separate antenna, not shown) may be used to transmit and receive radiofrequency signals. Of course, any other suitable method of communication between the implants 220 is within the scope of the invention including a physical connection between the implants 220 (such as a filament or wire) although wireless communication is preferred.

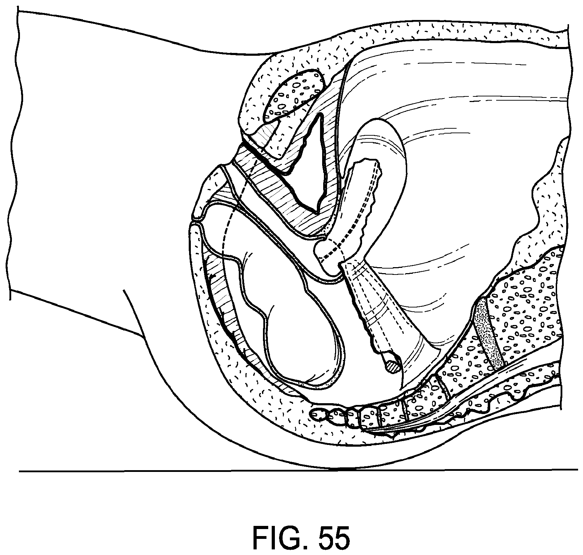

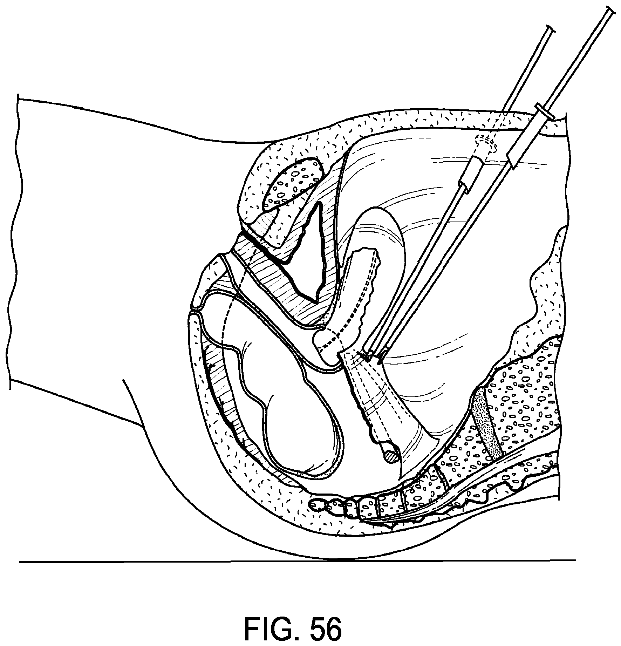

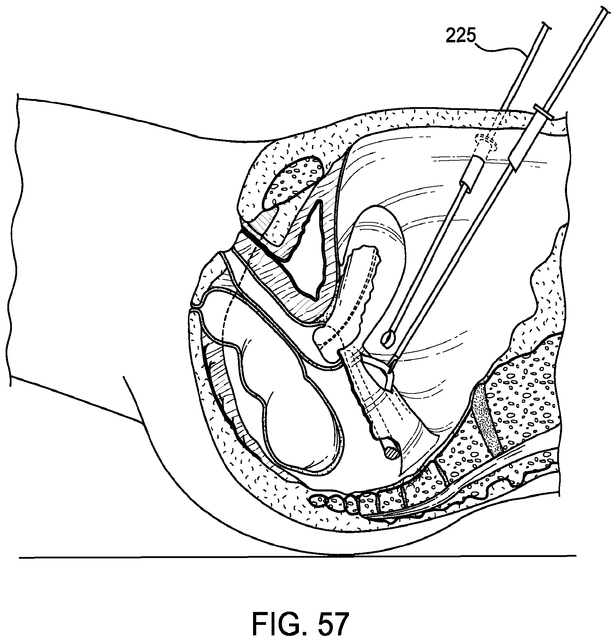

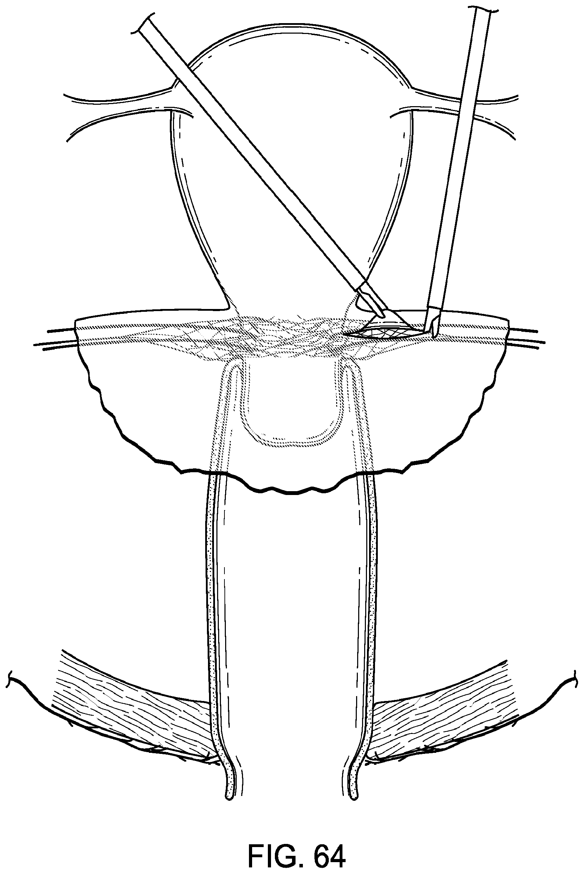

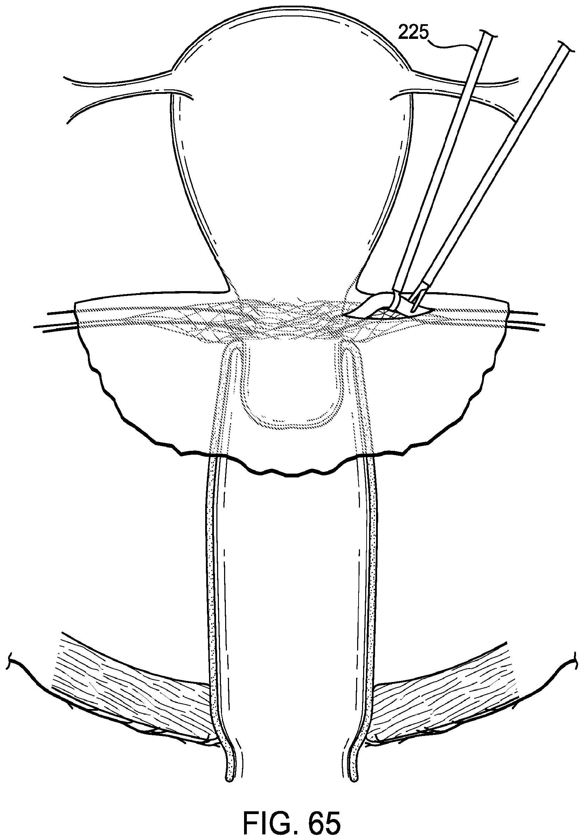

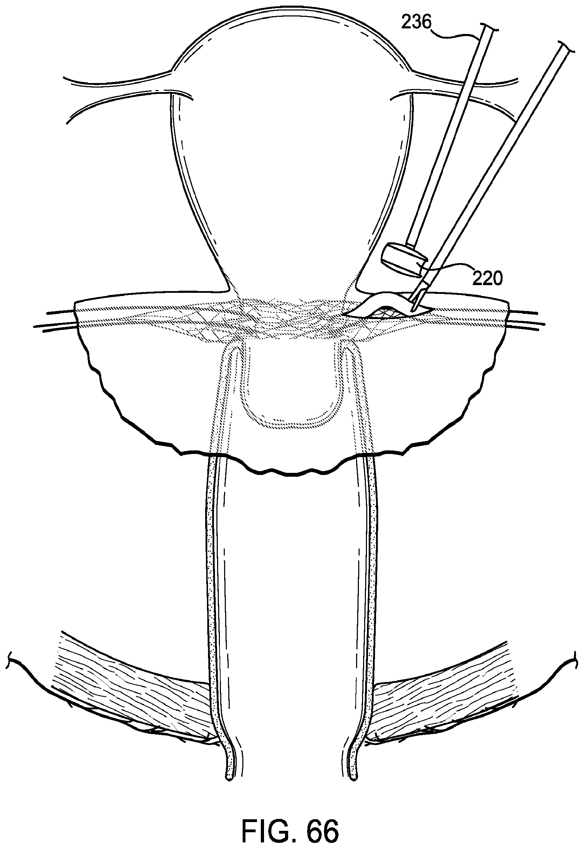

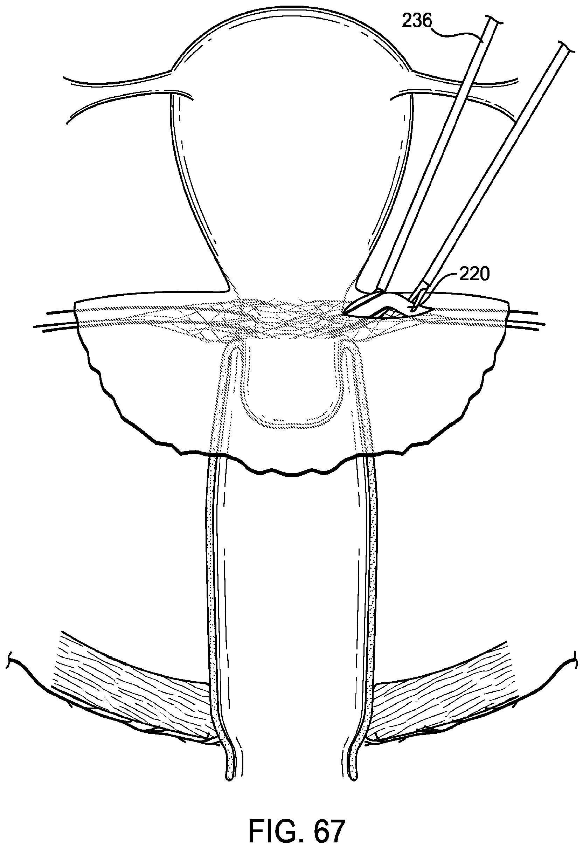

Referring now to Figures 55-67, methods of delivering the implant 220 are now described. The implant 220 may be placed using traditional and /or robotic assisted laparoscopic techniques. A female patient is placed under general anesthesia and positioned for gynecologist laparoscopy in dorsal lithotomy and Trendelenberg position as shown in Figure 55. Using laparoscopic and/or robotic technique with a camera and assisting port, the peritoneum over the intended portion of the uterosacral ligament is tented and an opening or window developed to gain retroperitoneal access as shown in Figure 56. The ligament is then elevated with a grasper and mobilized medially as shown in Figure 57.

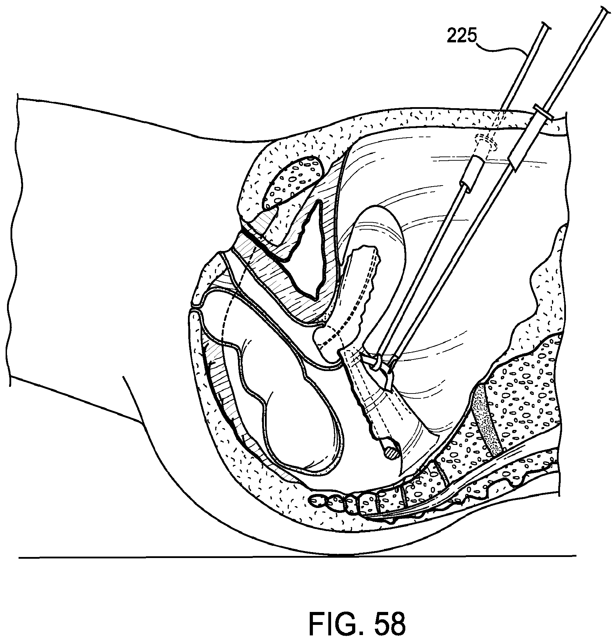

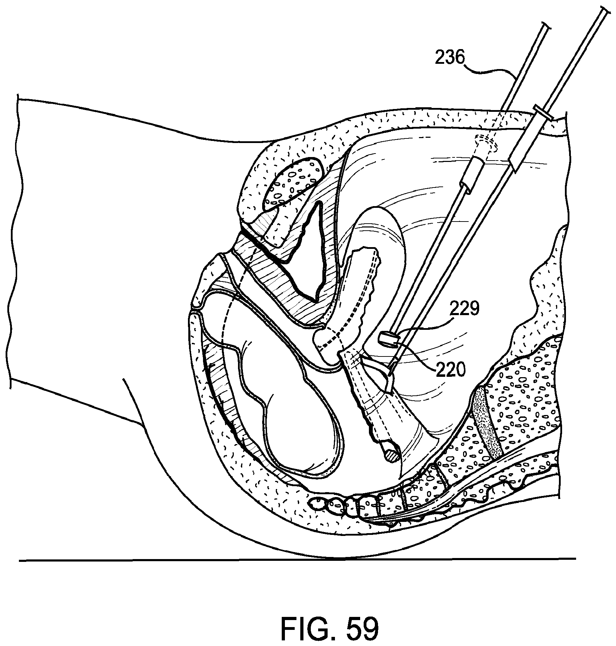

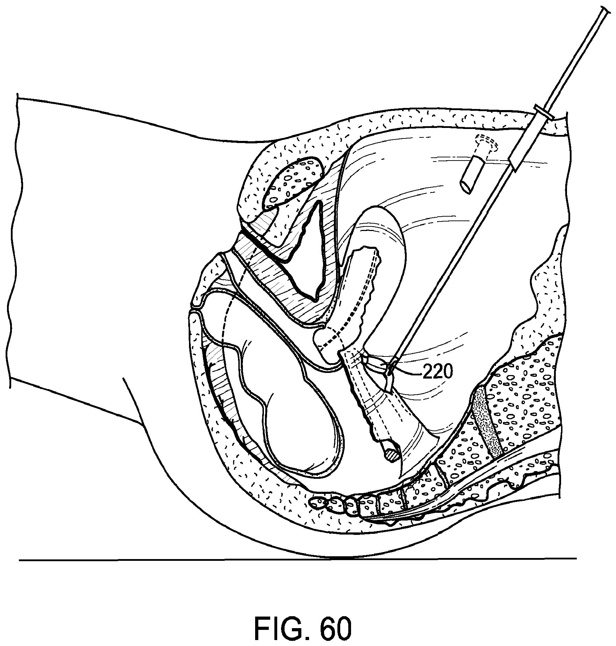

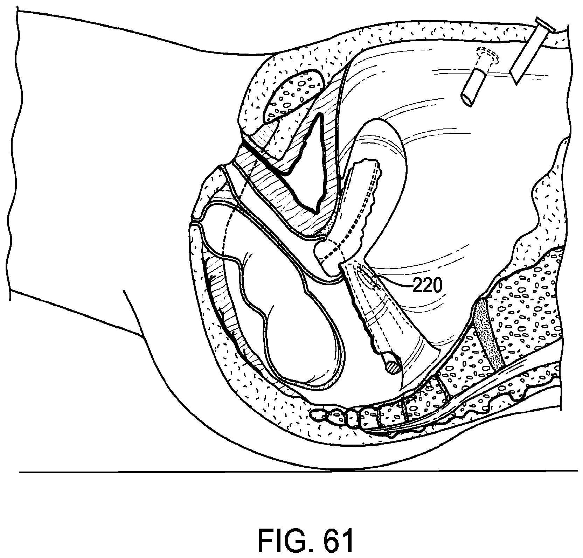

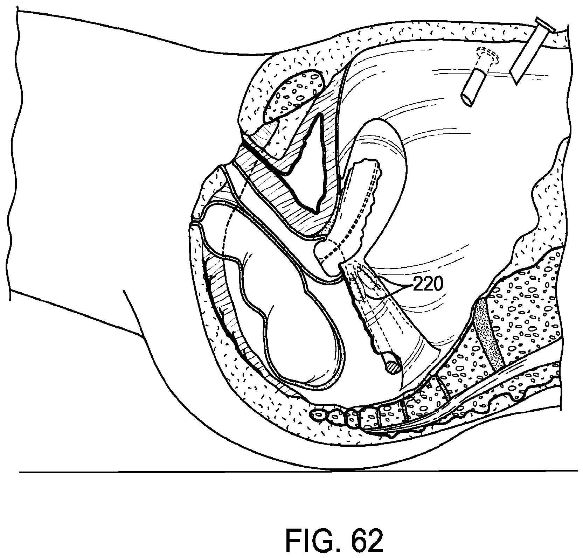

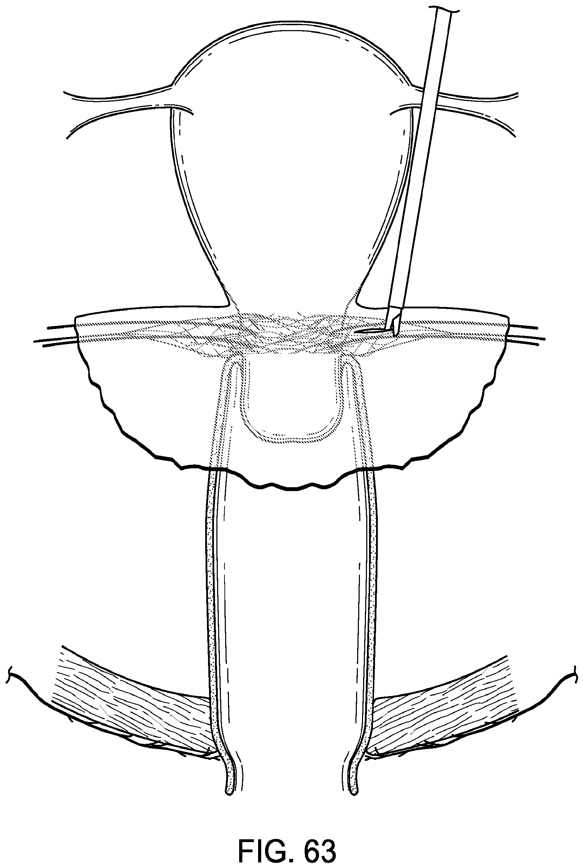

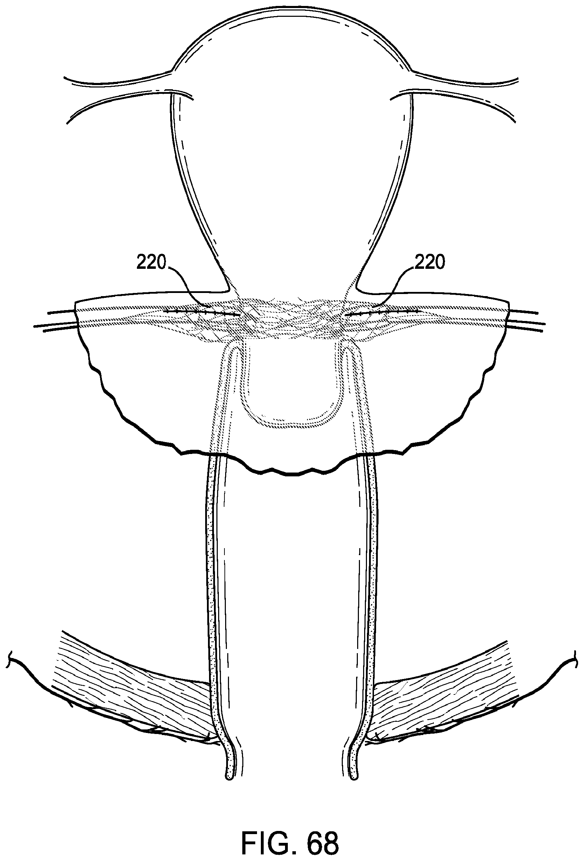

A ligament sizer 225 is introduced and the size of the ligament (circumference, diameter and/or thickness) is measured as shown in Figure 58. When the implant 220A is used, at least two measurements are taken to provide an appropriately shaped tapered throughhole 227 A. The sizer 225 may use any conventional method of determining thickness, for example, using magnetic, electromagnetic (not shown) objects to determine a spacing between the objects, The delivery tool 236, loaded with the implant 220 of proper size, is then introduced into the abdomen as shown in Figure 59. The delivery tool 236 is then used to position the ligament within the throughhole 227 of the implant 220. The delivery tool 236 may include a pivoting holder 229 to facilitate positioning the implant 220 around the ligament. The surgeon will actuate the movable jaw 235 to simply release the implant 220 if the implant 220 is held open by the delivery tool 236. Alternatively, the delivery tool 236 may clamp the implant 220 to the ligament using the movable jaw 235. Figure 60 shows the implant 220 secured to the ligament and Figure 61 shows the peritoneum closed. Figure 62 shows one implant secured to each uterosacral ligament. Figures 63-68 present an alternative view of the above-described procedure for placing the implant 220.

The implant 220 may be further secured to the ligament with permanent, monofilament sutures (not shown). Either end of the exposed longitudinal edges of the implant 220 may also include an arrow-like barb (not shown), attached to a fixed length of suture that is projected via an internal mechanism into the receiving opening on the opposing side of the throughhole 227. The arrow-like barb may be relatively large so that it becomes trapped and contained within the implant 220. The proximal and distal sutures may be delivered simultaneously with a fixed suture length. A properly fitted implant 220 will demonstrate two approximately 2 - 3 mm, exposed sutures on the medial surface. One or more suture(s) may be passed through the ligament to further prevent the implant 220 from traveling or slipping along the ligament. The implant 220 is then retroperitonealized surgically with absorbable suture. The profile of the implant 220 and the suture features are designed to prevent bowel adherence, in the case that the retroperitonealized implants become intraperitoneal.

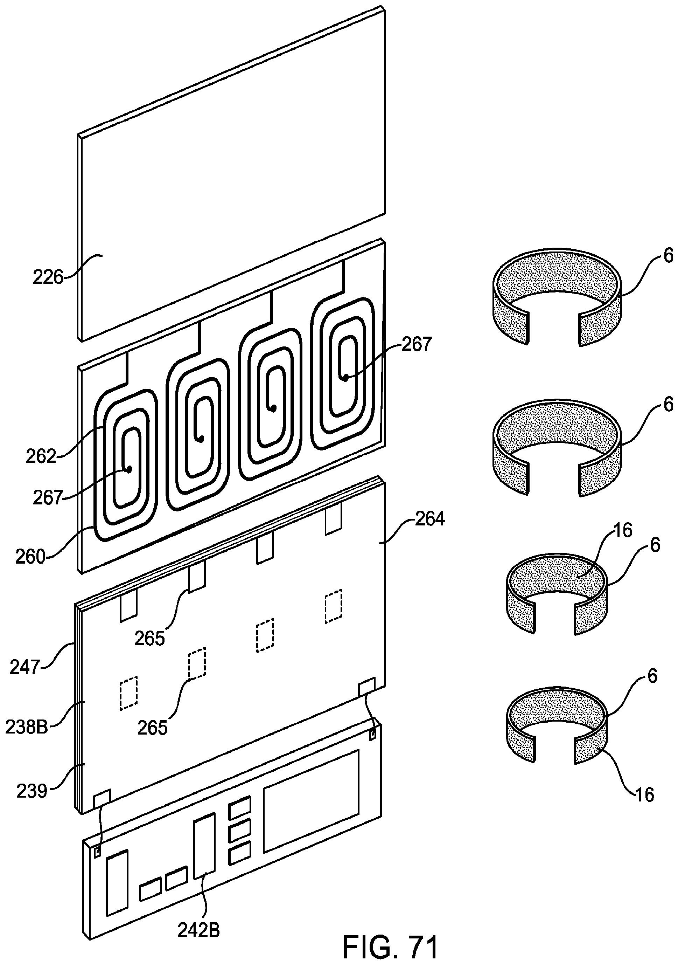

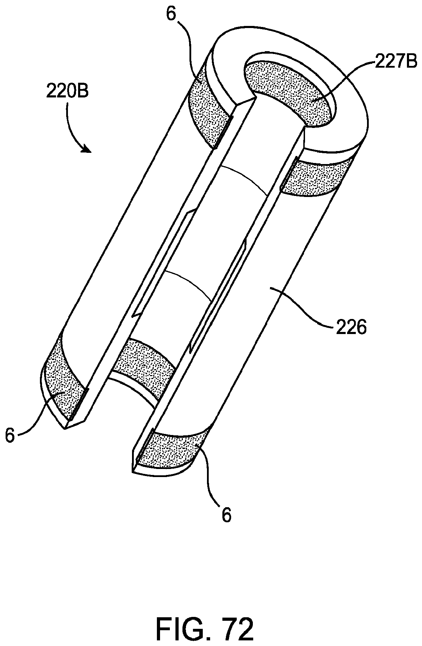

Referring to Figs. 71 and 72, another implant 220B is shown which may be used in the same manner as any of the other implants 220, 220A (including combinations with any of the devices 2A-N described herein) and all such uses are expressly incorporated here. For example, the implant 220B may be positioned with a uterosacral ligament positioned in a throughhole 227B. The implant 220B has a power source 238B which is preferably a capacitor 239 but may be the battery 240 (see Fig. 52) without departing from numerous aspects of the present invention. Battery powered implants, including rechargeable batteries, suffer from several disadvantages. First, battery life is often the limiting factor in implant life. Implants are also often limited in size due to size constraints for many implant applications. Unfortunately, a smaller battery obviously results in a shorter battery life. Rechargeable batteries may extend the life of a conventional implant, however, rechargeable batteries are still limited in the number of recharge cycles they can typically withstand. Replacing the battery (or removing the implant) typically requires another surgical procedure. Finally, batteries also contain potentially toxic materials, complicating the design, manufacture and regulatory tasks for an implantable medical device.

The capacitor 239 may be made of any suitable capacitor materials with more recently developed materials offering high energy densities comparable to a battery. Another advantage of the capacitor 239 is a very high cycle life. One distinct advantage of the capacitor 239 over a rechargeable battery is that the capacitor 239 can be recharged thousands, even tens of thousands, of times. One such material for capacitor 239 is made of layers of graphene and polymer 247. The implant 220B includes an induction element 260, such as an induction coil 262, printed, etched or otherwise deposited or formed on a first flexible substrate 264. The implant 220B has four induction elements 260. A control system 242B is formed on a second substrate 266. The capacitor 239, the control system 242B and an implant body 226 are formed into a roll and bonded together and sandwiched between the nerve stimulating elements 6 to form the implant 220B of Fig. 72. During operation, the control system 242B regulates discharge of the capacitor 239 to power the implant 220B and generate nerve stimulating impulses as described herein. The control system 242B may include switches (not shown) to selectively isolate or couple the induction coil 262 to the capacitor 239 as necessary. In one mode of operation, the capacitor 239 is either being charged or energy is drawn from the capacitor 239 to power the implant 220B. The induction coils 262 are each coupled to a rectifier 267 which supplies DC current to charge the capacitor 239.

The capacitor 239 may also facilitate design of a smaller implant 220B compared to conventional battery-powered implants since some capacitor materials can be recharged tens of thousands of times and, therefore, the capacitor 239 does not require high storage capacity since it can be reliably recharged thousands of times (unlike batteries which can withstand a much more limited number of recharges). To this end, the capacitor 239 may be sized to provide at least 2 days of power under peak operating conditions, and may be no more than 60 days, under peak operating conditions thereby maintaining a small size. Sizing the capacitor 239 in this manner will facilitate design of smaller implants 220B than a comparable battery-operated implant while also providing longer implant life since the capacitor 239 can be recharged many more times than typical rechargeable batteries.

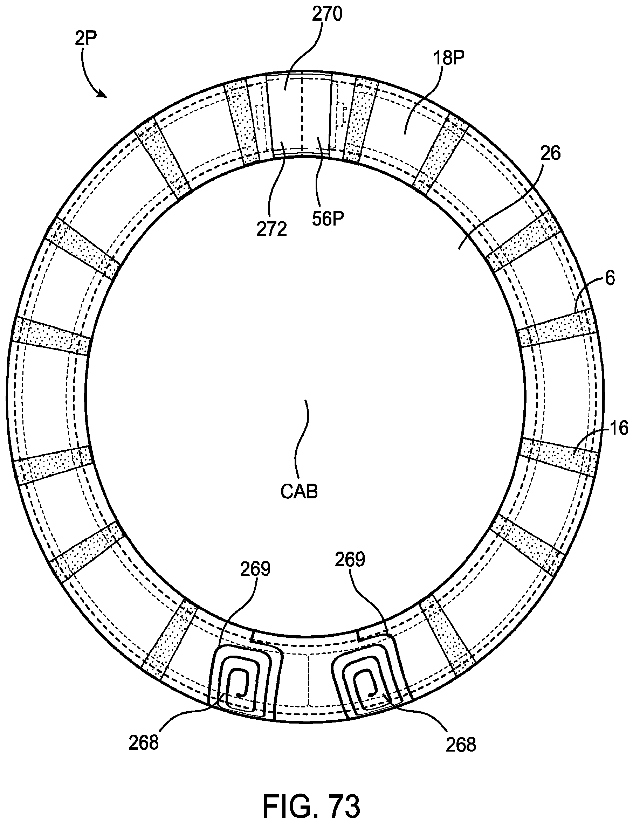

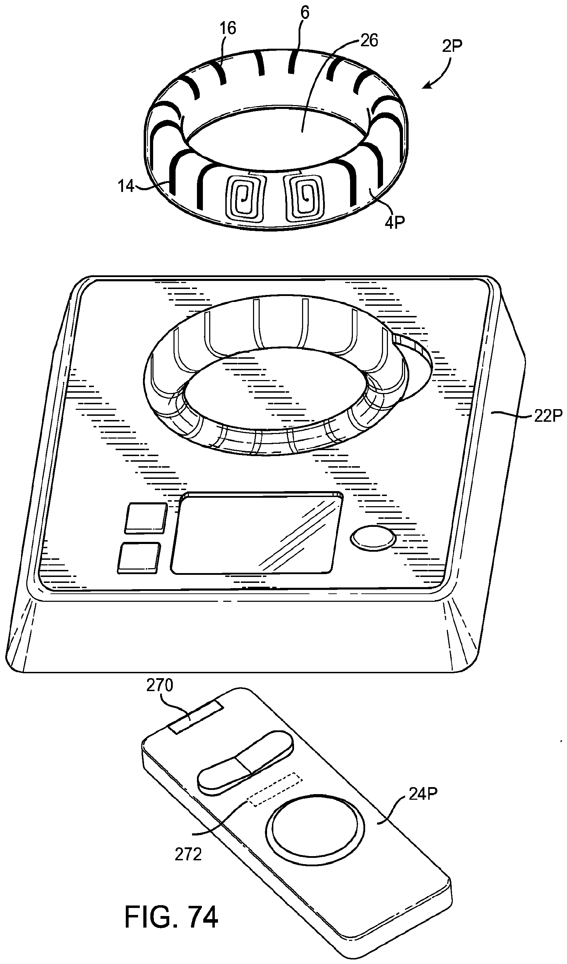

Referring now to Figs. 73 and 74, another device 2P designed to be positioned in the vagina (and in particular the vaginal fornices) is shown. The device 2P may also be used to charge the power source 238 of the implant 220B (such as the capacitor 239) of Figs. 71 and 72 but may be used with any other implanted medical device as well. The device 2P is similar to the devices 2, 2A-N and all uses and aspects of devices 2, 2A-N are incorporated here. The device 2P is similar to the device 2 except that two nerve stimulating elements 6 have each been replaced with a magnetic field creating element 268. The magnetic field creating element 268 includes one or more coils 269 configured to create a magnetic field, which impinges on the induction coils 262 of the implant 220B to charge the power source 238. The device 2P also includes nerve stimulating elements 6 which may be used in any manner described herein including all combinations described with the implants 220, 220A, 220B. A control system 18P regulates the current passing through the coils 269 in a conventional manner to provide a fluctuating, pulsing or otherwise changing magnetic field at the induction element 262 of the implant 220B. The induction elements 262 are electrically coupled to the capacitor 239 via contacts 265 on the front and back sides of the capacitor 239. The capacitor 239 is coupled to the implant control system 242B which draws electrical energy from the capacitor 239 and generates and delivers nerve stimulating therapy. The device 2P and control system 18P may operate in any manner described herein. Notably, all target plexuses may still be treated and targeted with the device 2P since adjacent nerve stimulating elements 6 are still capable of stimulating the target locations adjacent the magnetic field creating elements 268.

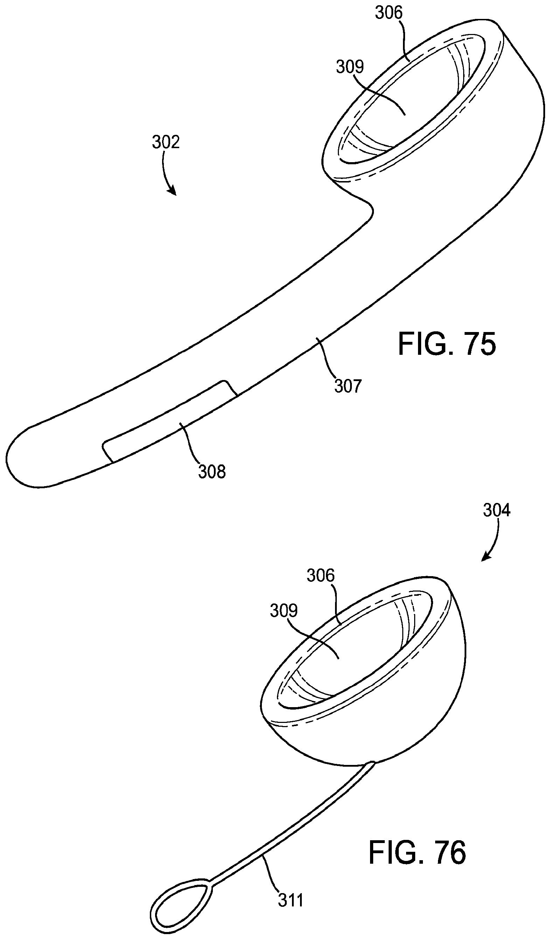

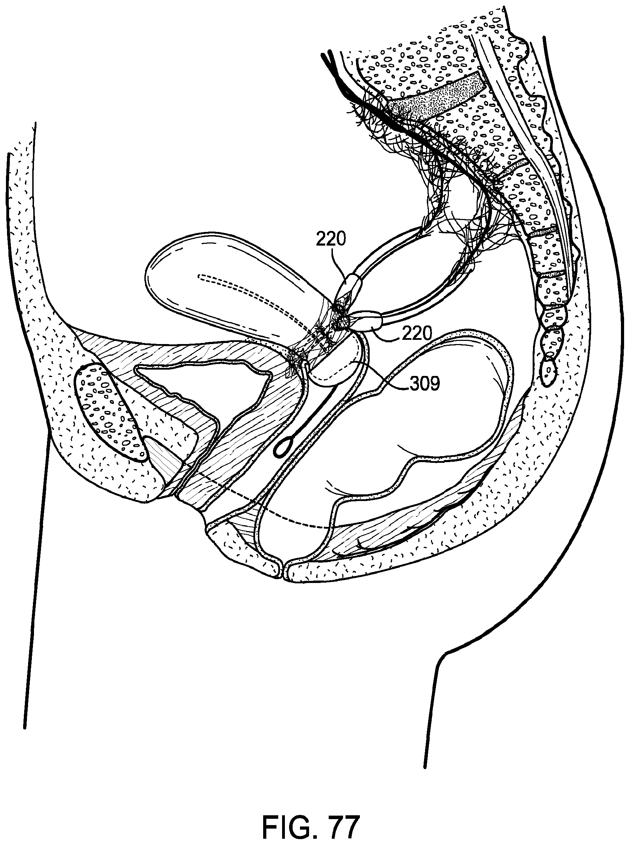

The implants 220, 220A, 220B may also be recharged with a separate device rather than using the nerve stimulating devices 2, 2A-P. Referring to Figs. 75 and 76, a first charger 302 and a second charger 304 are shown. The first and second chargers 302, 304 each include a magnetic field creating element 306 extending around a recess 309. The recess 309 may be part of a throughhole similar to the device 2 or may the recess 309 may be covered at the end of the recess 309 as shown. The first charger 302 has a handle 307 with an actuator 308 to turn the charger on and off. The second charger 304 includes a pull string 311 to aid in removing the second charger 304. The first and second chargers 302, 304 are substantially the same except for shape and the anatomy of the user may dictate which charger 302, 304 is more comfortable for the wearer. Fig. 77 shows the charger 306 in position to charge the power sources for the implants 220.

Referring again to the devices 2, 2A-P, the following discussion relates to methods and devices for confirming that the device 2, 2A-P is in the proper orientation, that is, that the device 2, 2A-P has not shifted so that the nerve stimulating elements 6 have changed position. The devices 2, 2A-2P are initially positioned by the user in a known starting orientation so that the nerve stimulating elements 6 are positioned adjacent their intended targets for stimulation. The marker 81 (see Fig. 9) may be useful when initially positioning and orienting the device 2P. The device 2P (or any of the other devices 2A-P) may also include a procedure to confirm that the device 2P has maintained the starting or intended orientation. To confirm that the device 2P is still positioned as intended, the device 2P is configured to assess the current orientation of device 2P to confirm that the orientation has not changed. The control system 18P may periodically assess the orientation of the device 2P or the user may transmit an instruction to the device 2P using the controller 24P to assess whether the device 2P has moved from the desired orientation relative to the cervix or the CVA. If the orientation has changed, the control system 18P uses the change in orientation information to reassign, as necessary, the nerve stimulating elements (based on the change in orientation) so that the nerve stimulating elements are again positioned adjacent the intended nerves and/or plexus(es) in the known orientation. In this manner, the device 2P may continue to operate normally without the need to reposition the device 2P as would otherwise be necessary.

As mentioned above, the control system 18P may assess the current orientation of the device 2P automatically and reassign the nerve stimulating elements 6 as necessary without user input. In one aspect, the device 2P may include a sensor 270 coupled to the control system 18P for sensing a parameter, which relates to the orientation of the device 2P. The control system 18P may sample the same parameter periodically to confirm that the device 2P has not moved. As mentioned above, the parameter may be an electrical signal received at one or more nerve stimulating elements 6 and emitted by another nerve stimulating element 6. Alternatively, the parameter may be another electrophysiological parameter such as tissue impedance or may simply be a measure of native electrical activity. Depending upon the particular parameter selected, the control system 18P may also sense and store the parameter relating to the starting or desired orientation for comparison to the parameter sensed for the current orientation.

Referring to Figure 74, the sensor 270 is mounted within a hub 56P and positioned at or near the midline 83 (see Fig. 9). The sensor 270 may be an independent element as shown or may be one or more of the nerve stimulating element(s) 6 as mentioned herein without departing from the scope of the invention. In one aspect, the sensor 270 may be configured to sense proximity to an object 272 which may be part of the controller 24P or may be independent of the controller 24P. The sensor 270 may be a magnetic sensor 270 and the object 272 may create a magnetic field, which the sensor 270 can remotely sense. The sensor 270 may, of course, operate in any other suitable manner such as a metal sensor 270 for use with the obj ect 272 being metallic, an ultraviolet light sensor which senses UV light emitted by the object 272, or the sensor 270 may be an RF antennae which receives RF signals emitted by the object 272. The object 272 may optionally be worn by the user, for example, the object 272 may be secured to clothing or an accessory (such as a belt) at a predetermined position. When the object is worn or carried by the user at the predetermined location the device 2P may periodically assess orientation automatically.

The object 272 may be carried by the controller 24P. The controller 24P is positioned at the predetermined location relative to the body of the user and the control system 18P is used to transmit an instruction to the device 2P to assess the current orientation of the device 2P. To this end, the user simply positions the controller 24P at the predetermined location, such as the belly button, and the controller 24P is then used to transmit an instruction to the device 2P to assess the orientation of the device 2P. The sensor 270 (such as the magnetic sensor) senses the object 272 and determines the orientation of the device based on the sensed data.

The sensor 270 may be a gravity sensor. Similar to other methods described above, the controller 24P initiates assessing device orientation by using the controller 24P to send an instruction to the device 2P to assess the orientation of the device 2P. Prior to sending the instruction to the device 2P, the user first positions herself in a predetermined body position, such as lying flat on her back. The controller 24P is then used to send a signal to instruct the control system 18P of the device 2P to assess the orientation. At this time, the sensor 270 determines the current orientation of the device 2P. The control system 18P may compare the sensor 270 gravity related data for the current orientation to data (either stored data or sensed and saved data) relating to the starting orientation. To this end, the control system 18P may store data relating to the starting orientation in the same manner. The user will assume the predetermined body position, such as lying down, and then uses the controller 24P to instruct the device 2P to use the sensor 270 to obtain sensor data relating to the starting or intended orientation for comparison to the sensor data relating to the current orientation. The sensor 270 and object 272 may also switch positions with the sensor 270 mounted to the controller 24P and the object 272 mounted to the device 2P. For example, the object 272 may simply be a metallic part of the hub 56P and the sensor 270 may be a metallic sensor 270 mounted to the controller 24P. The controller 24P receives information from the sensor 270 to assess the current orientation of the device 2P. The control system 18P transmits a signal to the device 2P to reassign the nerve stimulating elements 6 in accordance with the current orientation of the device 2P if necessary.

Assessing the orientation of the device 2P may also be accomplished by using one or more of the implants 220, 220A, 220B of the present invention like an internal marker or reference point. When the device 2P and implant 220B are used together, the implant 220B may emit a signal detected by the sensor 270 to assess orientation of the device 2P. The device 2P may also use two or more sensors 270 to triangulate the sensor data . Two or more nerve stimulating elements 6 may also be used to sense the electrical activity of one or more of the implants 220B. The device 2P may sense any suitable parameter relating to electrical activity of the implant 220B.

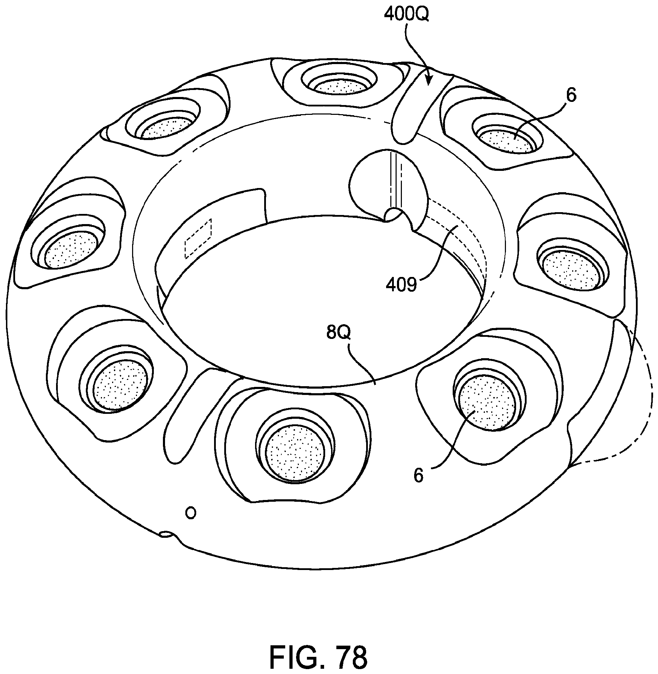

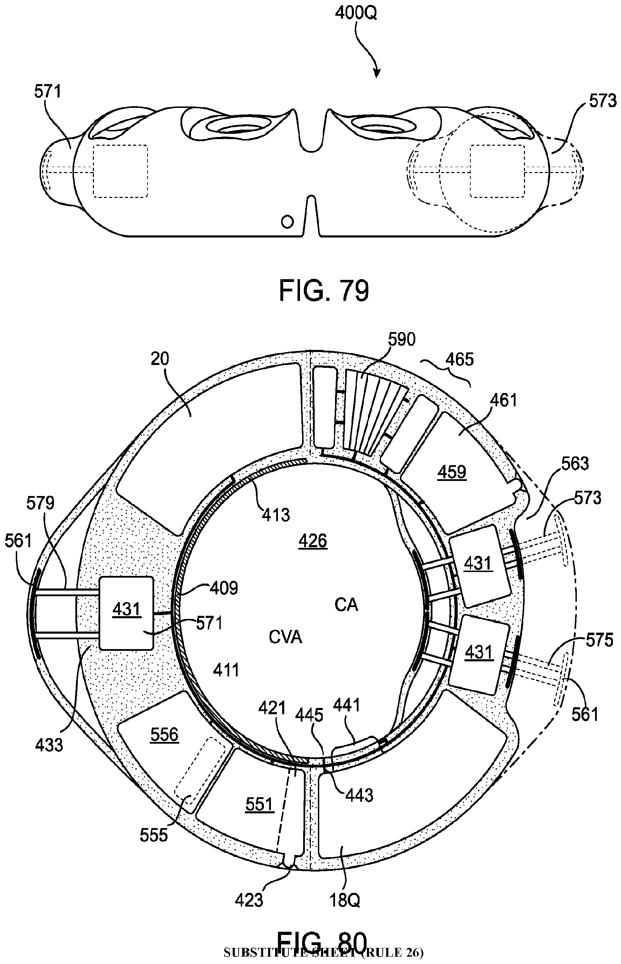

Referring to Figs. 78-83, another device 400Q is shown which is positioned in the vagina. The device 400Q includes a main body 4Q and nerve stimulating elements 6 positioned on exterior surface 8Q of the main body 4Q. The nerve stimulating elements 6 are controlled by control system 18Q and powered by battery 20 as described herein with reference to Fig. 80. The control system 18Q is coupled to each of the operating elements in a known manner.

As mentioned herein, the nerve stimulating elements 6 may also be sensors (see sensors 270 and accompanying description). For example, when the nerve stimulating elements 6 include conductive electrodes the nerve stimulating elements 6 may be used to obtain ECG data. For this purpose, electrodes on opposing sides of the ring may be selected. Various other sensors have also been previously described in connection with the devices of the present invention and those sensors are also incorporated here.

Embodiments will also provide features designed to optimize data sensing and monitoring, which further permits integration of data sensing with delivery of therapy. Both the device surface and the nerve stimulating elements may contain sensors and/or sensing electrodes for measuring such variables as acoustics, pressure, temperature, flow, acceleration, and pH. Semiconductor and fibre technologies, such as stretched Mylar, may both be used. Thus, the sensors may be of piezoelectric fibers, allowing some, or all, of the surface of the device to provide sensing information, different conductive layers measuring different signals. Capacitive pressure sensing may utilize bioMEMS.

The device 400Q may include additional sensors as now described. A blood pressure sensor 409 extends along the main body 4Q on a side facing the central opening. The blood pressure sensor 409 is in communication with the control system 18Q. The blood pressure sensor 409 may include a material 411, and may be an elongate strip of material 413, that can produce the required pressure information. For example, the material 411 may produce an electrical signal when deformed or produce a change in electrical resistance similar to a strain gauge. Piezoelectric materials, and other materials which convert vibration to an electrical signal, may also be useful for the blood pressure sensor 409 as any other known method of obtaining blood pressure. The blood pressure sensor 409 is coupled to the main body 4Q and may be embedded in a thin-walled portion of the main body 4Q. Furthermore, as will be further explained below, the blood pressure sensor 409 may also be separate from the main body 4Q and in wireless communication with the control system 18Q.

The device 400Q may also include other sensors. For example, a chemical sensor 421 (shown schematically in dotted-line), may sense an ion concentration, a pH or conductivity, for example. Known micro-devices can be incorporated for these uses. Each of these uses is expressly incorporated for the chemical sensor 421. The material is drawn through valve 423 which prevents material from entering under atmospheric pressure. The chemical sensor 421 may lead to a collection 551 as described further below.

The sensor data derived with any of the sensors described herein may be used to determine whether a condition exists that will trigger a corresponding therapeutic response from the device 400Q. For example, the nerve stimulating step using the elements 6 may be altered in response to the condition being met. As used herein, the term sensor may refer to devices that directly measure and transmit raw data or to devices that process the data and perform additional calculations and/or estimations to achieve the "sensor data" without departing from the scope of the invention or meaning of the word "sensor" or "sensor data" as used herein. The sensor data is assessed to determine whether the condition exists to provide a therapeutic response by the control system 18.

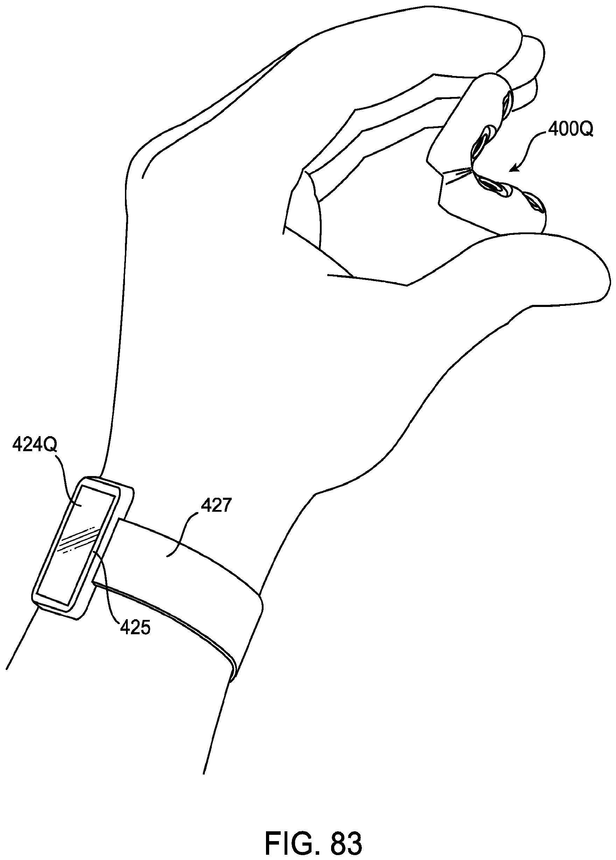

The sensor may be provided separate from the main body 4Q and positioned outside the vagina when the main body 4Q is positioned in the vagina. For example, a sensor 425, which may be a blood pressure sensor for example, may communicate raw data to the control system 18Q and is worn on the wrist as shown in Fig. 83. As used herein, such a relationship still falls within the term "controller" since the blood pressure sensor supplies data which controls the operation of the control system 18 at times. Referring to Fig. 83, for example, the controller 424Q may be worn around the wrist although any other suitable controller, such as a cell phone, may be used. The controller 424Q may operate in any manner and take any form of the controllers described herein.

The controller 424Q may include the sensor 423, such as the blood pressure sensor 425, as is known in the art. The controller 424Q may take any form described herein including a device worn on a limb, such as the arm and specifically wrist, and could be worn around the waist by simply lengthening a strap 427. Further, the controller 424Q may be used to control stimulation in any manner described herein. The controller 424Q is in wireless communication with control system 18Q. The sensor 425 may be coupled to at least one of the controller 424Q and to the main body 4Q. When coupled to the controller, the controller 424Q wirelessly communicates with the control system 18Q when the condition is determined to exist. As used herein, transmission of raw data to the control system 18Q shall be sufficient transmission of data when the data ultimately meets the condition warranting therapy in that the data still "controls" actions of the control system 18Q.

The device 400Q may include additional sensors such as a microphone 431. Magnetic actuators 433 are used to expand the main body 4Q, as described below, but may also act as microphones 431 to gather data by essentially listening or monitoring vibrations. For example, the microphone 431 may listen to respiration, heart beat, pulse and other suitable cardiovascular parameters. In a further specific example, the heart beat may be assessed to determine whether an arrhythmia condition exists. The condition could be related to heart rate, blood pressure, heart rate variability or any combination of factors with each factor being "related to" the condition.

The device 400Q may also include an optical sensor 441 which includes an emitter and receiver (not shown) to provide blood flow, pulse and heart rate data among other known values that may be derived from an optical sensor as is known in the art. A temperature sensor 443, in the form of a small wire probe 445, may also provide core body temperature. Other previously mentioned sensors, such as an accelerometer, may also be used here and such uses are expressly incorporated.

The sensors of the present invention may determine quantitative values or may be used to assess and determine changes in values rather than precise amounts. To this end, the condition may be based, at least in part, upon a baseline condition. Further, this condition may be based on a measurement taken at a prior time. For example, the condition may be determined with the measurement being taken by the same sensor related to the condition at a prior time. In another specific example, the sensor senses the heart beat for a period of time to obtain heartbeat data. The heart beat data is used to determine whether a heart beat variability of the heart beat data meets the condition. The heart beat variability may include a baseline for comparison from previously calculated heart beat variability at a prior time.

In another aspect of the device of 400Q, the device may include the collection reservoir 551 coupled to the main body 4Q. The collection reservoir 551 is configured to hold and collect material, including target material such as a microbe, from the vagina. The material may be assessed by the device 400Q itself. For example, the device 400Q may use the chemical sensor 421, such as a pH sensor. A low-pressure source 556 (at a pressure less than atmospheric pressure) is in fluid communication with the collection reservoir 551 to assist in drawing material into the material reservoir. A regulating valve 555 regulates pressure to control intake of the material into the collection reservoir 551. A small valve, such as a duck-bill valve, (not shown) prevents material from entering the collection reservoir under atmospheric pressure.

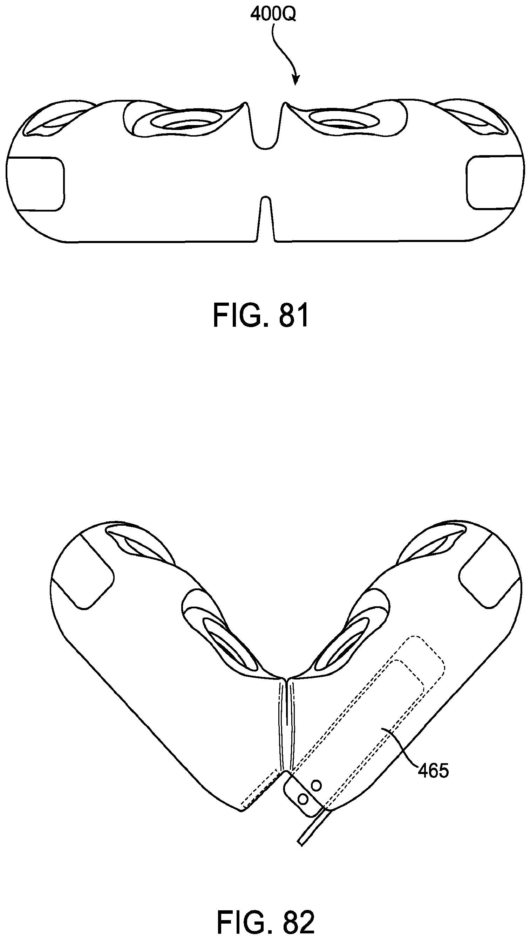

In still another aspect, a therapeutic material may also be delivered as now described based upon the sensor data meeting the particular condition. The therapeutic material 459 may be coupled to the main body 4Q of the device 400Q and held in a material reservoir 461. The therapeutic material 459 is delivered to the vagina by repositioning the device 400Q in the vagina once the therapeutic material is coupled to the main body 4Q such as by delivering the therapeutic material 459 into the material reservoir 461, by syringe or the like, or coupling the material reservoir 461 to the main body 4Q when the material reservoir 461 is removable and replaceable. Fig. 82 shows a module 465 that is removable and the material reservoir 461 and other components such as the battery and collection reservoir 551 may be formed in this manner for removal and replacement. The material reservoir 461 may be part of another module 465. A magnetic actuator 590 forces material out of the material reservoir. The magnetic actuator 590 may also be used to vibrate the main body 4Q. A valve, such as a duck bill valve, prevents material from entering the material reservoir.

The main body 4Q includes a first expandable portion 571 to increase a first dimension on the main body 4Q. The main body 4Q has a central axis CA with the first expandable portion 571 increasing the first dimension in a transverse direction to the central axis (or cervical axis CVA). Stated another way, the first expandable portion 571 increases a peripheral length of the main body 4Q when viewed along the central axis CA.

The main body 4Q may also have a second expandable portion 573 which also increases the first dimension, such as the peripheral length, when expanded. Alternatively, the second expandable portion may reduce the size of the central opening 426 when expanded. A third expandable portion 575 may be provided in parallel to the second expandable portion 573 to increase the force exerted. Furthermore, the second and third expandable portions 573, 575 may expand radially inward or outward.

The first, second and third expandable portions 571, 573, 575 are driven by magnetic actuators as is known in the art. The magnetic actuators each have two 579 arms extending to a common cross-member 561 supported by the two arms 579. The cross-members 561 may be embedded in a sidewall of the main body 4Q. A side slot 563 permits the second and third actuators 573, 575 to become slightly recessed to accommodate the opposing motion. Alternatively, the arms 579 may include telescoping elements (not shown) that permit the arms to extend rather than providing the side slot 563. The expandable portions 571, 573, 575 may be used to stretch the vaginal walls, which are believed to have therapeutic effect.

In another aspect of the present invention, the device 400Q may be used to deliver a therapeutic material. The material reservoir 461 containing the therapeutic material 459 may be coupled to the main body 4Q. Any one of the sensors described herein, such as the optical sensor, may be used to obtain sensor data. The sensor data is used to determine whether a condition exists from the data in the sensing step. If so, the therapeutic material 459 may be delivered. For example, the sensor may be a pH sensor, which determines whether a pH condition is met. The control system may control the delivery of the therapeutic material in appropriate amounts based upon, for example, the pH measurement by the pH sensor. One such clinical application for local (intravaginal) drug delivery would involve titrating the dose and delivery of vaginal estrogen to the pH of the vagina in postmenopausal women with atrophic vaginitis. In premenopausal women the vagina is relatively acidic. Healthy vaginal flora is rich with estrogen responsive Lactobacilli. Lactobacilli make lactic acid and keep the vaginal pH approximately 3.8 to 4.5. In menopause the flora changes, as estrogen dependent Lactobacilli fail to thrive. As a result the vaginal pH rises in menopause, generally above 5.0 and potentially to 7.5. 40% of postmenopausal women suffer from atrophic vaginitis symptoms. Vaginal hormone, such as estrogen, is an effective treatment. However, many women are hesitant to use even low dose estrogen out of concern for the known risks. Currently the dosing is not very individualizable, as standard doses are used. The vaginal device described could periodically assess vaginal pH and adjust estrogen dosing and delivery such that the user receives the lowest dose of estrogen required to maintain a healthy vaginal pH. In this manner, the pH of the vagina may be altered with the therapeutic material.

Thus, a few of the numerous applications of the insert and/or reservoir are now briefly provided. pH can be measured by a sensor and a rise in pH treated with dose-controlled estrogen. Cytology collection allows for study of cell biology, and application of therapeutic material. Collection of vaginal flora (that part of the microbiome that inhabits the vaginal cavity) allows for analysis of microbes and delivery of therapeutic material, for example related to infection.

In another embodiment of the closed-loop sensing and treatment of a parameter, the device described may treat systemic parameters. For example, the device may sense blood pressure (measured with a sensor on the controller or on the main body), heart rate or heart rate rhythm and dispense an appropriate amount of therapeutic material to alter that parameter. For example, the therapeutic material being selected to alter at least one of a heart rate and a heart rhythm. The parameters for treatment response would be based on a predetermined and programmed algorithm. The device could sense and interpret parameter response to the drug, assuring that dosing is appropriately titrated to response.

The vaginal wall is permeable via diffusion, paracellular tight junction mediated mechanisms, and receptor or vesicular transport mechanisms. Chemical polymers may also aid in macromolecule delivery through the vaginal wall. Advantages to vaginal delivery of systemic drugs include the absorptive wall, which is more permeable than external skin, has a rich arterial and venous blood supply, and avoids first-pass metabolism.

This ability of the device 2A-P to utilize sensors and other means to assess and automatically adjust orientation based on measured parameters allows for much wider use of sensors by the device to monitor bodily parameters. As mentioned above, sensors on the device 2A-P may be one or more of the nerve stimulating elements or may be independent elements. The sensors used may include some of the sensors mentioned in the above discussion of orientation, but may also include any other sensors, biosensors, and transducers known in the art. These sensors may communicate with other sensors on the device 2A-P itself, with sensors on an object which is independent of or integrated into the controller as described above (that controller being separate from or part of a user's personal device, such as a smartphone), and with sensors on other medical and computing devices. The sensors may include transducers, converting energy between forms.

This ability to compare and communicate sensor data provides numerous advantages. On the one hand, some important parameters are accessible from the location of the device 2A-P in the vaginal fornices, but not accessible from external devices, such as those worn by a user. In other words, the device 2A-P is uniquely positioned in the core of the body. Therefore, the device 2A-P has access to such measurements as core body temperature, and can sense tissue and bodily fluids in its internal bodily environment. On the other hand, there are other parameters measured by the device 2A-P, which can also be measured by other devices positioned internal or external to the body. For example, the user may be wearing a device with sensors, which can make measurements from an external location through the skin. This allows for comparing and triangulating data from device 2A-P to provide information beyond that available from the externally-worn device alone. Device 2A-P can also draw on other historical data. All data sensed by device 2A-P can be integrated into complementary diagnostic and therapeutic functions.

The control systems 242, 242B of the devices 2, 2A-2N and/or the handheld controller 24 may record data regarding treatment regimens including start and end times, nerve stimulating elements used, resistance, current, frequency, disruption of therapy and any changes made to a regimen. The user will also be able to record subjective and objective information, which will be used to adjust and tailor future regimens to her specific needs. For example, if she has a good response to a certain regimen she may note it at the end of a day or predetermined assessment period. In this case, the regimen will be maintained or she may opt to decrease stimulation intensity towards the threshold of benefit.

The user may also note when she experiences a side effect. By reviewing the treatment regimen (including current, frequency of stimulation, active electrodes and duration of therapy) employed during the time the side effect was experienced, she and her healthcare team will be able to change the stimulation setting or redirect therapy to another target structure altogether.

The user may record experiences using the controller 24 or via free text respond to periodic questions or standardized questionnaires and bladder diaries. All data will be recorded and downloadable to computers and portable devices, such as phones. In one aspect, the user's personal device (ie., phone) may reprogram the controller 24 and alter the regimen. If the patient desires and consents, this objective, subjective and regimen data may be transmitted electronically for remote assessment by healthcare providers or for research purposes.

The present invention may be used to treat a number of different conditions such as urge, frequency, nocturia, urge incontinence, stress incontinence, loss of urine without sensory awareness, bladder pain, urethral pain, urethral syndrome, urinary hesitancy, pelvic floor dyssynergia, interstitial cystitis, dysuria, overactive bladder, urinary retention, hesitancy, protracted urinary stream, dysmenorrhea, pelvic pain, pelvic venous congestion syndrome, endometriosis, irritable bowel syndrome, constipation, fecal urgency, fecal incontinence, rectal pain, pain with defecation, and anal pain. The conditions that may be treated extend to sympathetic tone, cardiovascular function, sexual function, and other conditions related to autonomic nerves. Of course, other uses of the present invention may become apparent without departing from the scope of the invention.

The present invention has been described in connection with preferred embodiments but it is understood that numerous modifications could be made to the preferred embodiments without departing from the scope of the invention. For example, the main body could be V-shaped or the nerve stimulating element could be a coil through which a current is passed to induce a magnetic field without departing from numerous aspects of the present invention.

## Claims

1. A system for stimulating nerves, comprising
a first body (226) having an outer surface, the first body having a first throughhole (227) sized to receive the uterosacral ligament;
a first nerve stimulating element (224) coupled to the first body;
a first power source (238) coupled to the first body; and
a first control system (242) coupled to the first power source and the first nerve stimulating element.

2. The system of claim 1, further comprising:
a second nerve stimulating element coupled to the body, the second nerve stimulating element positioned to apply nerve stimulus at a different position along a uterosacral ligament from the first nerve stimulating element when the first body is secured to the uterosacral ligament.

3. The system of claim 1, wherein:
the first nerve stimulating element is configured to stimulate nerves sufficiently to change a signal transmitted by the nerves to associated nerves and nerve endings.

4. The system of claim 1, wherein:
the first control system is configured to actuate the first nerve stimulating element at a different time from the second nerve stimulating element.

5. The system of claim 1, wherein:
the first body is movable from an open position to a locked position, the first body having a slot leading to the first throughhole when the first body is in the open position through which the uterosacral ligament is introduced.

6. The system of claim 5, wherein:
the slot reduces in size when the first body moves to the locked position so that the first body may be secured to the uterosacral ligament in the locked position.

7. The system of claim 5, wherein:
the first nerve stimulating element is positioned within the first throughhole so that the first nerve stimulating element is in contact with the uterosacral ligament.

8. The system of claim 1, further comprising:
a second nerve stimulating element;
the second nerve stimulating element being positioned along the outer surface of the first body.

9. The system of claim 1, wherein:
the first nerve stimulating element is an electrode.

10. The system of claim 1, wherein:
the first nerve stimulating element is configured to stimulate the middle rectal plexus when positioned in contact with a uterosacral ligament.

11. The system of claim 10, wherein:
the first nerve stimulating element stimulates the middle rectal plexus to treat at least one condition from the list of conditions consisting of fecal urge, bowel disorder, and incontinence.

12. The system of claim 10, further comprising:
a second nerve stimulating element coupled to the first body, the second nerve stimulating element being secured to the patient so that the second nerve stimulating element is positioned to stimulate the IHP;
wherein the first and second nerve stimulating elements stimulate the IHP and middle rectal plexus to treat a rectal condition. stimulating the IHP and the middle rectal plexus to treat rectal conditions.

13. The system of claim 1, wherein:
the first nerve stimulating element is configured to be positioned on the uterosacral ligament so that the first nerve stimulating element stimulates the hypogastric nerve.

## Patentansprüche

1. System zur Nervenstimulierung, umfassend:
einen ersten Körper (226) mit einer Außenfläche, wobei der erste Körper ein erstes Durchgangsloch (227) aufweist, das so bemessen ist, dass es das uterosakrale Ligament aufnimmt;
ein erstes nervenstimulierendes Element (224), das mit dem ersten Körper verbunden ist;
eine erste Energiequelle (238), die mit dem ersten Körper verbunden ist; und
ein erstes Steuersystem (242), das mit der ersten Energiequelle und dem ersten nervenstimulierenden Element verbunden ist.

2. Das System nach Anspruch 1, ferner umfassend:
ein zweites nervenstimulierendes Element, das mit dem Körper verbunden ist, wobei das zweite nervenstimulierende Element so positioniert ist, dass es einen Nervenreiz entlang eines uterosakralen Ligaments an einer anderen Stelle als das erste nervenstimulierende Element ausübt, wenn der erste Körper an dem uterosakralen Ligament befestigt ist.

3. Das System nach Anspruch 1, wobei:
das erste nervenstimulierende Element so konfiguriert ist, dass es Nerven ausreichend stimuliert, um ein von den Nerven an zugehörige Nerven und Nervenenden übertragenes Signal zu verändern.

4. Das System nach Anspruch 1, wobei:
das erste Steuersystem so konfiguriert ist, dass es das erste nervenstimulierende Element zu einem anderen Zeitpunkt als das zweite nervenstimulierende Element betätigt.

5. Das System nach Anspruch 1, wobei:
der erste Körper von einer offenen Position in eine verriegelte Position bewegbar ist, wobei der erste Körper, wenn sich der erste Körper in der offenen Position befindet, einen Schlitz aufweist, der zu dem ersten Durchgangsloch führt, durch das das uterosakrale Ligament eingeführt wird.

6. Das System nach Anspruch 5, wobei:
der Schlitz sich verkleinert, wenn sich der erste Körper in die verriegelte Position bewegt, so dass der erste Körper in der verriegelten Position an dem uterosakralen Ligament befestigt werden kann.

7. Das System nach Anspruch 5, wobei:
das erste nervenstimulierende Element innerhalb des ersten Durchgangslochs positioniert ist, so dass das erste nervenstimulierende Element in Kontakt mit dem uterosakralen Ligament steht.

8. Das System nach Anspruch 1, ferner umfassend:
ein zweites nervenstimulierendes Element;
wobei das zweite nervenstimulierende Element entlang der Außenfläche des ersten Körpers angeordnet ist.

9. Das System nach Anspruch 1, wobei:
das erste nervenstimulierende Element eine Elektrode ist.

10. Das System nach Anspruch 1, wobei:
das erste nervenstimulierende Element so konfiguriert ist, dass es den mittleren rektalen Plexus stimuliert, wenn es in Kontakt mit einem uterosakralen Ligament steht.

11. Das System nach Anspruch 10, wobei:
das erste nervenstimulierende Element den mittleren rektalen Plexus stimuliert, um mindestens eine Beschwerde aus der Liste der Beschwerden zu behandeln, die aus Stuhldrang, Darmstörung und Inkontinenz besteht.

12. Das System nach Anspruch 10, ferner umfassend:
ein zweites nervenstimulierendes Element, das mit dem ersten Körper verbunden ist, wobei das zweite nervenstimulierende Element am Patienten so befestigt ist, dass das zweite nervenstimulierende Element so positioniert ist, dass es den unteren hypogastrischen Plexus (UHP) stimuliert;
wobei die ersten und zweiten nervenstimulierenden Elemente den UHP und den mittleren Rektalplexus stimulieren, um eine rektale Beschwerde zu behandeln, wobei der UHP und der mittlere Rektalplexus stimuliert werden, um rektale Beschwerden zu behandeln.

13. Das System nach Anspruch 1, wobei:
das erste nervenstimulierende Element so konfiguriert ist, dass es auf dem uterosakralen Ligament positioniert wird, so dass das erste nervenstimulierende Element den hypogastrischen Nerv stimuliert.

## Revendications

1. Système de stimulation nerveuse comprenant
un premier corps (226) ayant une surface extérieure, le premier corps ayant un premier trou de passage (227) dimensionné pour recevoir le ligament utérosacré;
un premier élément de stimulation nerveuse (224) couplé au premier corps;
une première source d'énergie (238) couplée au premier corps; et
un premier système de commande (242) couplé à la première source d'énergie et au premier élément de stimulation nerveuse.

2. Le système de la revendication 1, comprenant également:
un second élément de stimulation nerveuse couplé au corps, le second élément de stimulation nerveuse étant positionné de manière à appliquer une stimulation nerveuse le long du ligament utérosacré à une position différente du premier élément de stimulation nerveuse lorsque le premier corps est fixé au ligament utérosacré.

3. Le système de la revendication 1, dans lequel:
le premier élément de stimulation nerveuse est configuré pour stimuler les nerfs suffisamment pour modifier un signal transmis par les nerfs aux nerfs associés et aux terminaisons nerveuses.

4. Le système de la revendication 1, dans lequel:
le premier système de commande est configuré pour actionner le premier élément de stimulation nerveuse à un moment différent du second élément de stimulation nerveuse.

5. Le système de la revendication 1, dans lequel:
le premier corps peut passer d'une position ouverte à une position verrouillée, le premier corps présentant une fente menant, lorsque le premier corps est en position ouverte, au premier trou de passage par lequel le ligament utéro-sacré est introduit.

6. Le système de la revendication 5, dans lequel :
la taille de la fente diminue lorsque le premier corps passe en position verrouillée de sorte que le premier corps peut être fixé au ligament utérosacré en position verrouillée.

7. Le système de la revendication 5, dans lequel :
le premier élément de stimulation nerveuse est positionné dans le premier trou de passage de sorte que le premier élément de stimulation nerveuse est en contact avec le ligament utérosacré.

8. Le système de la revendication 1, comprenant également :
un deuxième élément de stimulation nerveuse ;
le second élément de stimulation nerveuse étant positionné le long de la surface extérieure du premier corps.

9. Le système de la revendication 1, dans lequel :
le premier élément de stimulation nerveuse est une électrode.

10. Le système de la revendication 1, dans lequel :
le premier élément de stimulation nerveuse est configuré pour stimuler le plexus rectal moyen lorsqu'il est placé en contact avec un ligament utérosacré.

11. Le système de la revendication 10, dans lequel :
le premier élément de stimulation nerveuse stimule le plexus rectal moyen pour traiter au moins une affection parmi la liste d' affections comprenant le besoin impérieux de selles, les troubles intestinaux et l'incontinence.

12. Le système de la revendication 10, comprenant également :
un deuxième élément de stimulation nerveuse couplé au premier corps, le deuxième élément de stimulation nerveuse étant fixé au patient de sorte que le deuxième élément de stimulation nerveuse est positionné pour stimuler le plexus hypogastrique inférieur (PHI) ;
les premier et second éléments de stimulation nerveuse stimulant le PHI et le plexus rectal moyen pour traiter une affection rectale, en stimulant le PHI et le plexus rectal moyen pour traiter les affections rectales.

13. Le système de la revendication 1, dans lequel :
le premier élément de stimulation nerveuse est configuré pour être positionné sur le ligament utérosacré de sorte que le premier élément de stimulation nerveuse stimule le nerf hypogastrique.
